(19) Europäisches Patentamt
European Patent Office
Office européen des brevets

(11) EP 3 558 222 B1

(12) EUROPEAN PATENT SPECIFICATION

(45) Date of publication and mention of the grant of the patent:
**03.04.2024 Bulletin 2024/14**

(21) Application number: **17804878.1**

(22) Date of filing: **24.11.2017**

(51) International Patent Classification (IPC):
***A61K 8/26*** (2006.01) ***A61K 8/39*** (2006.01)
***A61K 8/41*** (2006.01) ***A61Q 19/00*** (2006.01)
***A61K 8/895*** (2006.01) ***A61K 8/06*** (2006.01)
***A61Q 1/02*** (2006.01) ***A61K 8/86*** (2006.01)
***A61K 8/897*** (2006.01) ***A61K 8/02*** (2006.01)

(52) Cooperative Patent Classification (CPC):
**A61Q 19/00; A61K 8/0241; A61K 8/064; A61K 8/26; A61K 8/39; A61K 8/416; A61K 8/86; A61K 8/895; A61K 8/897; A61Q 1/02;**
A61K 2800/624

(86) International application number:
**PCT/EP2017/080311**

(87) International publication number:
**WO 2018/114214 (28.06.2018 Gazette 2018/26)**

(54) **WATER-IN-OIL EMULSION COMPRISING A PARTICULAR EMULSIFYING SYSTEM, A LIPOPHILIC CLAY, AND AN ORGANOPOLYSILOXANE ELASTOMER POWDER COATED WITH A SILICONE RESIN**

WASSER-IN-ÖL EMULSION ENTHALTEND EINE SPEZIELLE KOMBINATION UAS EMULGATOREN, EINEN LIPOPHILEN TON UND EIN PULVER AUS ORGANOSILIXANELASTOMER ÜBERZOGEN MIT EINEM SILIKONHARZ

ÈMULSION EAU-DANS-HUILE COMPRENANT UN SYSTÈME D'EMULSIFIANTS PARTICULIER, UNE ARGILE LIPOPHILIQUE ET UNE POUDRE D'ORGANOSILOXANE ÉLASTOMÈRE RECOUVERTE AVEC UNE RÉSINE DE SILICONE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2016 FR 1662975**

(43) Date of publication of application:
**30.10.2019 Bulletin 2019/44**

(73) Proprietor: **L'OREAL**
**75008 Paris (FR)**

(72) Inventors:
- **VALVERDE, Elodie**
  **94152 Chevilly la Rue (FR)**
- **LI, Hong**
  **94152 Chevilly la Rue (FR)**

(74) Representative: **L'Oreal**
**Service D.I.P.I.**
**9, rue Pierre Dreyfus**
**92110 Clichy (FR)**

(56) References cited:
**WO-A1-2016/030420 WO-A2-2014/170865**
**FR-A1- 2 977 486**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

The present invention relates to a composition, in particular comprising a physiologically acceptable medium, in particular for coating keratin materials, more particularly for making up and/or caring for keratin materials, such as the skin, in the form of a water-in-oil emulsion comprising:

a) a continuous oily phase, and
b) at least one discontinuous aqueous phase dispersed in said oily phase;
c) at least one emulsifying system comprising:

i) at least one fatty acid ester of a polyol and
ii) at least one polymer of polyoxyethylenated fatty acid ester of glycol; and

d) at least one lipophilic clay; and
e) at least one organopolysiloxane elastomer powder coated with a silicone resin.

The invention also relates to a process for coating keratin materials, more particularly for making up and/or caring for keratin materials, such as the skin, characterized in that it comprises the application to the keratin materials of a composition as defined previously.

Water-in-oil emulsions are highly sought-after galenical forms, due to the ease with which they are applied to the skin and their sensory properties in cosmetics in the care field, in particular in anti-sun compositions, in anti-ageing compositions and in makeup, such as foundation.

Water-in-oil emulsions do not always exhibit, on the one hand, storage stability that is entirely satisfactory equally at low temperatures (i.e: 4°C), at ambient temperature (i.e: 25°C) and at high temperatures (i.e: 45°C and 55°C) and, on the other hand, sensory properties that are entirely satisfactory, such as softness, a non-tacky effect, a non-greasy effect, the absence of an unpleasant film sensation after application, a feeling of lightness after application, or a good glide.

Patent application WO-A-2016/030420 describes cosmetic makeup or skincare compositions in the form of a water-in-oil emulsions comprising 0.5% to 1% relative to the total weight of the composition of an organo-modified clay (i.e. hectorites modified by a C10 to C22 ammonium chloride), 5% to 12% of a silicone elastomer powder coated with a silicone resin and a silicone surfactant.

The objective of the present invention is therefore to find novel water-in-oil emulsions which exhibit good storage stability equally at low temperatures (i.e: 4°C), at ambient temperature (i.e: 25°C) and at high temperatures (i.e: 45°C) and also good sensory properties.

The Applicant has discovered, surprisingly, that this objective can be achieved with a composition, in particular comprising a physiologically acceptable medium, in particular for coating keratin materials, more particularly for making up and/or caring for keratin materials, such as the skin, in the form of a water-in-oil emulsion containing at least:

a) a continuous oily phase, and
b) at least one discontinuous aqueous phase dispersed in said oily phase;
c) at least one emulsifying system comprising:

i) at least one fatty acid ester of a polyol and
ii) at least one polymer of polyoxyethylenated fatty acid ester of glycol; and

d) at least one lipophilic clay; and
e) at least one organopolysiloxane elastomer powder coated with a silicone resin.

This discovery forms the basis of the invention.

Thus, according to one of its aspects, the present invention relates to a composition, in particular comprising a physiologically acceptable medium, in particular for coating keratin materials, more particularly for making up and/or caring for keratin materials, such as the skin, in the form of a water-in-oil emulsion comprising:

a) a continuous oily phase, and
b) at least one discontinuous aqueous phase dispersed in said oily phase;
c) at least one emulsifying system comprising:

i) at least one fatty acid ester of a polyol and

ii) at least one polymer of polyoxyethylenated fatty acid ester of glycol; and

d) at least one lipophilic clay; and
e) at least one organopolysiloxane elastomer powder coated with a silicone resin.

The invention also relates to a process for coating keratin materials, more particularly for making up and/or caring for keratin materials, such as the skin, characterized in that it comprises the application to the keratin materials of a composition as defined previously.

## DEFINITIONS

In the context of the present invention, the term "keratin material" is intended to mean in particular the skin (body, face, area around the eyes, eyelids).
The term "physiologically acceptable" is intended to mean compatible with the skin and/or its integuments, which has a pleasant colour, odour and feel, and which does not cause any unacceptable discomfort (stinging, tautness) liable to discourage the consumer from using this composition.
For the purposes of the present invention, "water-in-oil emulsion", also referred to as inverse emulsion, is intended to denote any composition constituted of a continuous oily phase in which the aqueous phase is dispersed in the form of droplets so as to observe a macroscopically homogeneous mixture with the naked eye.
For the purposes of the present invention, the term "emulsifying surfactant" is intended to mean an amphiphilic surfactant compound, i.e. one which has two parts of different polarity. Generally, one is lipophilic (soluble or dispersible in an oily phase). The other is hydrophilic (soluble or dispersible in water). The emulsifying surfactants are characterized by the value of their HLB (Hydrophilic Lipophilic Balance), the HLB being the ratio between the hydrophilic part and the lipophilic part in the molecule. The term "HLB" is well known to those skilled in the art and is described, for example, in "The HLB system. A time-saving guide to Emulsifier Selection" (published by ICI Americas Inc., 1984). For the emulsifying surfactants, the HLB generally ranges from 3 to 8 for the preparation of W/O emulsions. The HLB of the surfactant(s) used according to the invention may be determined via the Griffin method or the Davies method.

## FATTY ACID ESTERS OF A POLYOL

According to the invention, the term "fatty acid ester of a polyol" is intended to mean an ester of a fatty acid (or fatty acid polymer) and of a polyol in which the fatty acid comprises a $C_6$-$C_{22}$ and preferably $C_{16}$-$C_{20}$ alkyl chain and the polyol is chosen from glycerol, a polyglycerol and sorbitan, and mixtures thereof. The fatty acid may also be in a polymeric form, as is the case for polyhydroxystearic acid (12-hydroxystearic acid polymer).
According to a particular embodiment, the fatty acid ester of a polyol is a $C_{16}$-$C_{20}$ fatty acid ester of glycerol and/or sorbitan, and mixtures thereof.
As examples of linear- or branched-chain $C_{16}$-$C_{20}$ fatty acids, mention may be made of stearic acid, isostearic acid, lauric acid, myristic acid, palmitic acid. An example of a $C_{16}$-$C_{20}$ fatty acid polymer that may be mentioned is poly(12-hydroxystearic acid).
Preferably stearic acid, isostearic acid, or poly(12-hydroxystearic acid) and mixtures thereof will be used.
Polyglycerols are understood to mean compounds having formula:

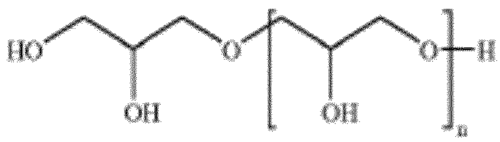

in which the degree of condensation n ranges from 1 to 11, preferably from 2 to 6 and even more preferentially from 3 to 6.
According to a specific embodiment, the ester of a fatty acid and a polyol contains 2 to 10 moles (units) of polyols, preferably 2 to 4 moles of polyols, in particular 2 to 4 units of glycerol or a mixture of polyglycerols (glycerol, di-, tri-, tetra-, penta-, oligoglycerols).
Even more preferentially, the ester of a fatty acid and a polyol contains 4 mol (or units) of polyol, in particular 4 mol (or units) of glycerol.
According to one preferred embodiment, said ester of a fatty acid and a polyol is further a fatty acid ester of a dicarboxylic acid having from 2 to 16 carbon atoms, preferably from 8 to 14 carbon atoms, such as azelaic acid, sebacic acid, dodecanedioic acid, and preferably sebacic acid (C10), and a polyol.
As examples of esters of fatty acids and polyols that can be used in the composition of the invention, mention

may be made of esters of isostearic acid and polyols and mixtures thereof, in particular the esters of isostearic acid and glycerol and/or of sorbitan, such as for example polyglycerolated (4 moles) isostearate (INCI name: Polyglyceryl-4 Isostearate) sold under the name Isolan GI34® by Goldschmidt, polyglycerolated (3 mol) diisostearate sold under the name Lameform TGI® by the company Cognis; polyglycerolated distearate (2 mol) sold under the name Emalex PGSA® by the company Nihon emulsion; polyglycerolated (10 mol) monoisostearate sold under the name Nikkol Decaglyn 1-IS® by the company Nihon Surfactant (INCI name: Polyglyceryl-10 isostearate); polyglyceryl-4 diisostearate polyhydroxystearate sebacate sold under the name Isolan GPS® by the company Goldschmidt; the mixture of sorbitan isostearate and glyceryl isostearate, such as the product sold under the name Arlacel 986® by the company ICI, the mixture of sorbitan isostearate and polyglyceryl (3 mol) isostearate sold under the name Arlacel 1690® by the company Unigema, and mixtures thereof.

As fatty acid esters of polyglycerol that are preferred according to the invention, mention may be made in particular of: polyglycerolated (4 mol) I isostearate (INCI name: Polyglyceryl-4 isostearate) sold under the name Isolan GI34® by Goldschmidt, polyglyceryl-4 diisostearate polyhydroxystearate sebacate sold under the name Isolan GPS® by Goldschmidt, the mixture of sorbitan isostearate and polyglyceryl (3 mol) isostearate sold under the name Arlacel 1690® by the company Unigema, and mixtures thereof.

According to one preferred embodiment of the invention, the fatty acid ester of a polyol according to the invention is an ester of poly(12-hydroxystearic acid) and of dicarboxylic acids obtained by esterification of a mixture of polyglycerol with (i) a polyhydroxystearic acid, with from 1 to 10, preferably from 2 to 8, even more preferentially from 2 to 5 polyglycerol units (preferably 4 units), (ii) linear or branched aliphatic dicarboxylic acids containing from 2 to 16 carbon atoms, preferably from 4 to 14 carbon atoms (preferably sebacic acid), and (iii) saturated or unsaturated, linear or branched fatty acids containing from 6 to 22 carbon atoms, preferably from 16 to 20 carbon atoms (preferably isostearic acid).

Advantageously, the degree of esterification of the polyglyceryl mixture is between 20% and 40%, preferably between 40% and 70%.

Such poly(12-hydroxystearic acid) esters of polyglyceryl are described in application US 2005/0031580.

According to one preferred embodiment, the fatty acid ester of a polyol is an ester of poly(12-hydroxystearic acid) and of dicarboxylic acids obtained by esterification of a mixture of polyglycerol with (i) a polyhydroxystearic acid, with from 2 to 5 polyglycerol units, (ii) linear or branched aliphatic dicarboxylic acids containing 4 to 14 carbon atoms, and (iii) saturated or unsaturated, linear or branched fatty acids containing from 16 to 20 carbon atoms.

Preferably, the fatty acid ester of a polyol is an ester of polyhydroxystearic acid and of dicarboxylic acids obtained by esterification of a mixture of polyglycerol with (i) a polyhydroxystearic acid, with from 2 to 5 polyglycerol units (preferably 4 units), (ii) linear or branched aliphatic dicarboxylic acids containing 4 to 14 carbon atoms (preferably sebacic acid) and (iii) saturated or unsaturated, linear or branched fatty acids containing from 16 to 20 carbon atoms (preferably isostearic acid).

Even more preferably, use will be made, in the composition of the invention, of polyglyceryl-4 diisostearate polyhydroxystearate sebacate of formula:

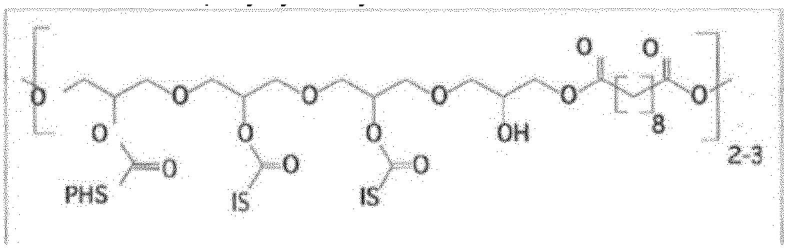

in which PHS denotes polyhydroxystearic acid and IS denotes isostearic acid and INCI name: Polyglyceryl-4 Diisostearate/Polyhydroxystearate/Sebacate.

Such a compound is prepared according to application US 2005/0031580 and sold under the name Isolan GPS® by the company Evonik Goldschmidt (Degussa).

The fatty acid ester of a polyol is preferably present in the composition in contents ranging from 0.1 % to 5% by weight, more preferentially from 0.1 % to 2% by weight and more particularly from 0.1% to 0.75% by weight relative to the total weight of the composition.

## POLYOXYETHYLENATED GLYCOL FATTY ACID ESTER POLYMER

The fatty acid ester of said polymer is polyhydroxylated. In particular, this polymer is a block polymer, preferably of ABA structure, comprising poly(hydroxylated ester) blocks and polyethylene glycol (or polyoxyethylene) blocks.

The fatty acid ester of said emulsifying polymer as defined above generally has a chain comprising from 12 to

20 carbon atoms and preferably from 14 to 18 carbon atoms. The esters may be chosen in particular from oleates, palmitates or stearates.

The polyethylene glycol blocks of said emulsifying polymer as defined above preferably comprise from 4 to 50 mol of ethylene oxide and more preferably from 20 to 40 mol of ethylene oxide.

A compound that is particularly suitable for preparing the compositions of the invention is polyethylene glycol dipolyhydroxystearate containing 30 EO, sold under the trade name Cithrol DPHS-SO-(MV)® by the company Croda and having the INCI name: PEG-30 Dipolyhydroxystearate.

The polymer of polyoxyethylenated fatty acid ester of glycol is preferably present in the composition in contents ranging from 0.1% to 5% by weight, more preferentially from 0.1 % to 2% by weight and more particularly from 0.1 % to 1 % by weight, relative to the total weight of the composition.

## LIPOPHILIC CLAY

The composition in accordance with the invention comprises at least one lipophilic clay.

The term "lipophilic clay" is intended to mean any clay that is liposoluble or lipodispersible in the oily phase of the composition.

The clay denotes a material based on hydrated silicates and/or aluminosilicates, of lamellar structure.

The clays can be natural or synthetic and they are rendered lipophilic by treatment with an alkylammonium salt, such as a $C_{10}$ to $C_{22}$ ammonium chloride, in particular stearalkonium chloride or distearyldimethylammonium chloride.

They may be chosen from bentonites, in particular bentonites, hectorites and montmorillonites, beidellites, saponites, nontronites, sepiolites, biotites, attapulgites, vermiculites and zeolites.

They are preferably chosen from hectorites and bentonites.

According to one particularly preferred form, use will be made of a lipophilic clay chosen from hydrophobically modified bentonites and hydrophobically modified hectorites, in particular modified with a $C_{10}$ to $C_{22}$ quaternary ammonium chloride, such as:

- a bentonite modified with stearalkonium chloride, such as the commercial products sold under the name Claytone AF®, Garamite VT®, Tixogel® LG-M, Tixogel® MP 250 Tixogel® VZ and Tixogel® VZ-V XR, by the company BYK Additives Inc; or the commercial products sold under the name Viscogel® B3, Viscogel® B4, Viscogel® B7, Viscogel® B8, Viscogel® ED, Viscogel® GM, Viscogel® S4 and Viscogel® SD by the company Bentec S.P.A;
- a bentonite modified with stearalkonium chloride in the presence of at least propylene carbonate and of at least one oil, such as the commercial products Dub Velvet Gum® from the company Stearineries Dubois Fils, Myglyol GEL T® from the company Cremer Oleo, Tixogel® CGT 6030, Tixogel® DBA 6060, Tixogel® FTN, Tixogel® FTN 1564, Tixogel® IPM, Tixogel® LAN, Tixogel® LAN 1563 by the company BYK Additives Inc;
- a hectorite modified with distearyldimethylammonium chloride (INCI name: Disteardimonium Hectorite) such as, for example, that sold under the name Bentone® 38V by the company Elementis Specialities;
- a hectorite modified with distearyldimethylammonium chloride in the presence of at least propylene carbonate or triethyl citrate and of at least one oil, such as the products sold under the name Bentone® Gel DOA V, Bentone® Gel EUG V, Bentone® Gel IHD V, Bentone® Gel ISD V, Bentone® Gel MIO V® Bentone® Gel PTM V® Bentone® SS-71 V, Bentone® VS-5 PC V, Bentone® VS-5 by the company

Elementis Specialities; the commercial products sold under the name Creagel Bentone CPS/Hectone CPS, Creagel Bentone ID/Hectone ID from the company Creations Couleurs; the commercial products sold under the name NS Gel DM1®, NS Gel PTIS®, NS MGel 1152® from the company Next Step Laboratories Stop.

The lipophilic clay(s) are present in the composition in concentrations ranging preferably from 0.1% to 5% by weight and more preferentially from 0.1% to 1% by weight relative to the total weight of the composition.

## ORGANOPOLYSILOXANE ELASTOMER POWDER COATED WITH SILICONE RESIN

The composition according to the invention comprises at least one organopolysiloxane elastomer powder coated with silicone resin, in particular silsesquioxane resin, as described, for example, in Patent US 5 538 793.

The term "organopolysiloxane elastomer" or "silicone elastomer" is intended to mean a supple, crosslinked, deformable organopolysiloxane with viscoelastic properties and in particular with the consistency of a sponge or a supple sphere. Its modulus of elasticity is such that this material withstands deformation and has a limited ability to extend and to contract. This material is capable of regaining its original shape after stretching.

Such elastomer powders are sold under the names KSP-100®, KSP-101®, KSP-102®, KSP-103®, KSP-104® and KSP-105® by the company Shin-Etsu, and have the INCI name: vinyl dimethicone/methicone silsesquioxane crosspolymer.

Mention may also be made of:

- organopolysiloxane elastomer powders coated with silicone resin, such as powders of hybrid silicone functionalized with fluoroalkyl groups, in particular sold under the name KSP-200® by the company Shin-Etsu and having the INCI name: Trifluoropropyl Dimethicone/ Vinyl Trifluoropropyldimethicone/Silsesquioxane Crosspolymer; or
- powders of hybrid silicones functionalized with phenyl groups, in particular sold under the name KSP-300® by the company Shin-Etsu and having the INCI name: Diphenyl Dimethicone/ Vinyl Diphenyl Dimethicone/Silsesquioxane Crosspolymer, and mixtures thereof.

According to one advantageous embodiment, the compositions according to the invention comprise an organopolysiloxane elastomer powder coated with silsesquioxane resin, having the INCI name Vinyl Dimethicone/Methicone Silsesquioxane Crosspolymer, such as those sold under the names KSP-100®.

The organopolysiloxane elastomer powder(s) coated with silicone resin may be present in a content ranging from 0.1% to 10% by weight, preferably from 0.5% to 5% by weight, more particularly from 0.5% to 2% by weight relative to the total weight of said composition.

## AQUEOUS PHASE

The aqueous phase comprises water and optionally water-soluble or water-miscible ingredients, such as water-soluble solvents.

A water that is suitable for use in the invention may be a floral water such as cornflower water and/or a mineral water such as Vittel water, Lucas water or La Roche Posay water and/or a spring water.

In the present invention, the term "water-soluble solvent" denotes a compound that is liquid at ambient temperature and water-miscible (miscibility in water of greater than 50% by weight at 25°C and atmospheric pressure).

The water-soluble solvents that may be used in the composition of the invention may also be volatile.

Among the water-soluble solvents that may be used in the composition in accordance with the invention, mention may be made in particular of lower monoalcohols having from 1 to 5 carbon atoms such as ethanol and isopropanol, glycols having from 2 to 8 carbon atoms such as ethylene glycol, propylene glycol, 1 ,3-butylene glycol and dipropylene glycol, $C_3$ and $C_4$ ketones and $C_2$-$C_4$ aldehydes.

The aqueous phase is preferably present in a concentration of at least 20% by weight, preferably ranging from 30% to 60% by weight, more particularly from 40% to 50% by weight relative to the total weight of said composition.

## OILY PHASE

The composition of the invention comprises a discontinuous oily phase. Said phase is liquid (in the absence of structuring agent) at ambient temperature (20-25°C). It is organic and water-immiscible.

The oily phase (or fatty phase) of the compositions according to the invention comprises at least one oil. It may be constituted of a single oil or of a mixture of several oils.

The term "oil" is intended to mean any fatty substance in liquid form at ambient temperature (20-25°C) and at atmospheric pressure. These oils may be of plant, mineral or synthetic origin.

According to one embodiment, the oils are chosen from the group constituted of hydrocarbon-based oils, silicone oils, fluoro oils and mixtures thereof.

The term "oil" is intended to mean a fatty substance which is liquid at ambient temperature (25°C) and atmospheric pressure (760 mmHg, i.e. $10^5$ Pa). The oil may be volatile or non-volatile.

Within the meaning of the present invention, "silicone oil" is intended to mean an oil comprising at least one silicon atom and in particular at least one Si-O group, and more particularly an organopolysiloxane.

The term "fluoro oil" is intended to mean an oil comprising at least one fluorine atom.

The term "hydrocarbon-based oil" is intended to mean an oil mainly containing hydrogen and carbon atoms and optionally one or more functions chosen from hydroxyl, ester, ether and carboxylic functions.

For the purposes of the invention, the term "volatile oil" is intended to mean any oil that is capable of evaporating on contact with the skin in less than one hour, at ambient temperature and atmospheric pressure. The volatile oil is a volatile cosmetic compound which is liquid at ambient temperature and which in particular has a non-zero vapour pressure, at ambient temperature and atmospheric pressure, which in particular has a vapour pressure ranging from 0.13 Pa to 40 000 Pa ($10^{-3}$ to 300 mmHg), in particular ranging from 1.3 Pa to 13 000 Pa (0.01 to 100 mmHg) and more particularly ranging from 1.3 Pa to 1300 Pa (0.01 to 10 mmHg).

The term "non-volatile oil" is intended to mean an oil that remains on the skin or the keratin fibre at ambient temperature and atmospheric pressure for at least several hours, and that in particular has a vapour pressure of less

than $10^{-3}$ mmHg (0.13 Pa).

The total concentration of oily phase of the composition of the invention preferably ranges from 40% to 95% by weight, more particularly ranging from 50% to 70% by weight, relative to the total weight of the composition.

Volatile oils

According to one embodiment, the oily phase of the compositions of the invention comprises at least one volatile oil. The oily phase of the compositions of the invention may comprise a mixture of several volatile oils.

The volatile oils may be hydrocarbon, silicone or fluoro oils.

The volatile oils may be chosen from hydrocarbon-based oils having from 8 to 16 carbon atoms, and in particular branched $C_8$-$C_{16}$ alkanes (also referred to as isoparaffins or isoalkanes), such as isododecane (also referred to as 2,2,4,4,6-pentamethylheptane), isodecane, isohexadecane and, for example, the oils sold under the trade names Isopar® or Permethyl®.

As volatile hydrocarbon-based oil, mention may also be made of linear $C_9$-$C_{17}$ alkanes, such as dodecane ($C_{12}$) and tetradecane ($C_{14}$), sold respectively under the references PARAFOL® 12-97 and PARAFOL® 14-97 (Sasol) and such as the alkanes obtained according to the process described in international application WO 2007/068371 A1, such as the mixture of undecane ($C_{11}$) and tridecane ($C_{13}$).

According to one embodiment, the oily phase of the compositions of the invention comprises at least one volatile silicone oil.

As volatile silicone oils, use may also be made of volatile silicones such as, for example, volatile linear or cyclic silicone oils, in particular those having a viscosity of less than or equal to 8 centistokes (cSt) ($8 \times 10^{-6}$ $m^2/s$), and in particular having from 2 to 10 silicon atoms and in particular from 2 to 7 silicon atoms, these silicones optionally comprising alkyl or alkoxyl groups having from 1 to 10 carbon atoms. As volatile silicone oil of use in the invention, mention may in particular be made of dimethicones with viscosities of 5 and 6 cSt, octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

More particularly, as volatile silicone oil, mention may be made of the linear or cyclic silicone oils containing from 2 to 7 silicon atoms, these silicones optionally comprising alkyl or alkoxyl groups containing from 1 to 10 carbon atoms.

As volatile fluoro oil, mention may be made, for example, of nonafluoromethoxybutane or perfluoromethylcyclopentane, and mixtures thereof.

According to a particular form of the invention, the oily phase comprises at least one non-cyclic volatile silicone oil.

Non-cyclic volatile silicone oils

The non-cyclic volatile silicone oils according to the invention are preferably chosen from:

- the non-cyclic linear silicones of formula (II):

$$R_3SiO-(R_2SiO)_n-SiR_3 \qquad (II)$$

  in which R, which may be identical or different, denotes:
- a saturated or unsaturated hydrocarbon-based radical, containing from 1 to 10 carbon atoms, preferably from 1 to 6 carbon atoms, optionally substituted with one or more fluorine atoms or with one or more hydroxyl groups, or
- a hydroxyl group,

  one of the radicals R possibly being a phenyl group,
  n is an integer ranging from 0 to 8, preferably ranging from 2 to 6 and better still ranging from 3 to 5, the silicone compound of formula (II) containing at most 15 carbon atoms.

- the branched silicones of formula (III) or (IV) below:

$$R_3SiO-[(R_3SiO)RSiO]-(R_2SiO)x-SiR_3 \qquad (III)$$

$$[R_3SiO]_4Si \qquad (IV)$$

in which R, which may be identical or different, denotes:

- a saturated or unsaturated hydrocarbon-based radical, containing from 1 to 10 carbon atoms, optionally substituted with one or more fluorine atoms or with one or more hydroxyl groups, or
- a hydroxyl group,

one of the radicals R possibly being a phenyl group,
x is an integer ranging from 0 to 8,
the silicone compound of formula (III) or (IV) containing at most 15 carbon atoms.

Preferably, for the compounds of formulae (II), (III) and (IV), the ratio between the number of carbon atoms and the number of silicon atoms is between 2.25 and 4.33.
The silicones of formulae (II) to (IV) may be prepared according to the known processes for synthesizing silicone compounds.
Examples of non-cyclic volatile silicone of use according to the invention are indicated below; these silicones may be used alone or in a mixture.
Among the silicones of formula (II) that may be mentioned are:

a) the following disiloxanes (L2):

hexamethyldisiloxane, in particular sold under the name DC 200 Fluid 0.65 cst by Dow Corning
1,3-di-tert-butyl-1,1,3,3-tetramethyldisiloxane;
1,3-dipropyl-1,1,3,3-tetramethyldisiloxane;
heptylpentamethyldisiloxane;
1,1,1-triethyl-3,3,3-trimethyldisiloxane;
hexaethyldisiloxane;
1,1,3,3-tetramethyl-1,3-bis(2-methylpropyl)disiloxane;
pentamethyloctyld isiloxane;
1,1,1-trimethyl-3,3,3-tris(1-methylethyl)disiloxane;
1-butyl-3-ethyl-1,1,3-trimethyl 3-propyldisiloxane;
pentamethylpentyldisiloxane;
1-butyl-1,1,3,3-tetramethyl-3-(1-methylethyl)d isiloxane;
1,1,3,3-tetramethyl-1,3-bis(1-methylpropyl)disiloxane;
1,1,3-triethyl-1,3,3-tripropyldisiloxane;
(3,3-dimethylbutyl)pentamethyldisiloxane;
(3-methylbutyl)pentamethyldisiloxane;
(3-methylpentyl)pentamethyldisiloxane;
1,1,1-triethyl-3,3-dimethyl-3-propyldisiloxane;
1-(1,1-dimethylethyl)-1,1,3,3,3-pentamethyldisiloxane;
1,1,1-trimethyl-3,3,3-tripropyld isiloxane;
1,3-dimethyl-1,1,3,3-tetrakis(1-methylethyl)disiloxane;
1,1-d ibutyl-1,3,3,3-tetramethyldisiloxane;
1,1,3,3-tetramethyl-1,3-bis(1-methylethyl)disiloxane;
1,1,1,3-tetramethyl-3,3-bis(1-methylethyl)disiloxane;
1,1,1,3-tetramethyl-3,3-dipropyldisiloxane;
1,1,3,3-tetramethyl-1,3-bis(3-methylbutyl)disiloxane;
butylpentamethyldisiloxane;
pentaethylmethyldisiloxane;
1,1,3,3-tetramethyl-1,3-d ipentyld isiloxane;
1,3-dimethyl-1,1,3,3-tetrapropyldisiloxane;
1,1,1,3-tetraethyl-3,3-dimethyldisiloxane;
1,1,1 -triethyl-3,3,3-tripropyld isiloxane;
1 ,3-dibutyl-1, 1 ,3,3-tetramethyldisiloxane; hexylpentamethyldisiloxane;

b) the following trisiloxanes (L3):

octamethyltrisiloxane, in particular sold under the name Xiameter PMX-200 Silicone Fluid 1CS by the company Dow Corning
3-pentyl-1,1,1,3,5,5,5-heptamethyltrisiloxane;
1-hexyl-1,1,3,3,5,5,5-heptamethyltrisiloxane;

1,1,1,3,3,5,5-heptamethyl-5-octyltrisiloxane;
1,1,1,3,5,5,5-heptamethyl-3-octyltrisiloxane, sold in particular under the name Silsoft 034 by the company OSI;
1,1,1,3,5,5,5-heptamethyl-3-hexyltrisiloxane, sold in particular under the name DC 2-1731 by the company Dow Corning;
1,1,3,3,5,5-hexamethyl-1,5-dipropyltrisiloxane;
3-(1-ethylbutyl)-1,1,1,3,5,5,5-heptamethyltrisiloxane;
1,1,1,3,5,5,5-heptamethyl-3-(1-methylpentyl)trisiloxane;
1,5-diethyl1,1,3,3,5,5-hexamethyltrisiloxane;
1,1,1,3,5,5,5-heptamethyl-3-(1-methylpropyl)trisiloxane;
3-(1,1-d imethylethyl)-1,1,1,3,5,5,5-heptamethyltrisiloxane;
1,1,1,5,5,5-hexamethyl-3,3-bis(1-methylethyl)trisiloxane;
1,1,1,3,3,5,5-heptamethyl-1,5-bis(1-methylpropyl)trisiloxane;
1,5-bis(1,1-dimethylethyl)-1,1,3,3,5,5-hexamethyltrisiloxane;
3-(3,3-dimethylbutyl-1,1,1,3,5,5,5-heptamethyltrisiloxane;
1,1,1,3,5,5,5-heptamethyl-3-(3-methylbutyl)trisiloxane;
1,1,1,3,5,5,5-heptamethyl-3-(3-methylpentyl)trisiloxane;
1,1,1,3,5,5,5-heptamethyl-3-(2-methylpropyl)trisiloxane;
1-butyl-1,1,3,3,5,5,5-heptamethyltrisiloxane;
1,1,1,3,5,5,5-heptamethyl-3-propyltrisiloxane;
3-isohexyl-1,1,1,3,5,5,5-heptamethyltrisiloxane;
1,3,5-triethyl 1,1,3,5,5-pentamethyltrisiloxane;
3-butyl-1,1,1,3,5,5,5-heptamethyltrisiloxane;
3-tert-pentyl-1,1,1,3,5,5,5-heptamethyltrisiloxane;
1,1,1,5,5,5-hexamethyl-3,3-dipropyltrisiloxane;
3,3-diethyl-1,1,1,5,5,5-hexamethyltrisiloxane;
1,5-dibutyl-1,1,3,3,5,5-hexamethyltrisiloxane;
1,1,1,5,5,5-hexaethyl-3,3-dimethyltrisiloxane;
3,3-dibutyl-1,1,1,5,5,5-hexamethyltrisiloxane;
3-ethyl-1,1,1,3,5,5,5-heptamethyltrisiloxane;
3-heptyl-1,1,1,3,5,5,5-heptamethyltrisiloxane;
1-ethyl-1,1,3,3,5,5,5-heptamethyltrisiloxane;

c) the following tetrasiloxanes (L4):

decamethyltetrasiloxane;
1,1,3,3,5,5,7,7-octamethyl-1,7-dipropyltetrasiloxane;
1,1,1,3,3,5,7,7,7-nonamethyl-5-(1-methylethyl)tetrasiloxane;
1-butyl-1,1,3,3,5,5,7,7,7-nonamethyltetrasiloxane;
3,5-diethyl-1,1,1,3,5,7,7,7-octamethyltetrasiloxane;
1,3,5,7-tetraethyl-1,1,3,5,7,7-hexamethyltetrasiloxane;
3,3,5,5-tetraethyl-1,1,1,7,7,7-hexamethyltetrasiloxane;
1,1,1,3,3,5,5,7,7-nonamethyl-7-phenyltetrasiloxane;
3,3-diethyl-1,1,1,5,5,7,7,7-octamethyltetrasiloxane;
1,1,1,3,3,5,7,7,7-nonamethyl-5-phenyltetrasiloxane;

d) the following pentasiloxanes (L5):

dodecamethylpentasiloxane;
1,1,3,3,5,5,7,7,9,9-decamethyl-1,9-dipropylpentasiloxane;
3,3,5,5,7,7-hexaethyl-1,1,1,9,9,9-hexamethyl pentasiloxane;
1,1,1,3,3,5,7,7,9,9,9-undecamethyl 5-phenylpentasiloxane;
1-butyl 1,1,3,3,5,5,7,7,9,9,9-undecamethylpentasiloxane;
3,3-diethyl 1,1,1,5,5,7,7,9,9,9-decamethylpentasiloxane;
1,3,5,7,9-pentaethyl-1,1,3,5,7,9,9-heptamethylpentasiloxane;
3,5,7-triethyl-1,1,1,3,5,7,9,9,9-nonamethylpentasiloxane;
1,1,1-triethyl-3,3,5,5,7,7,9,9,9-nonamethylpentasiloxane;

e) the following hexasiloxanes (L6):

1-butyl1,1,3,3,5,5,7,7,9,9,11,11,11-tridecamethylhexasiloxane;
3,5,7,9-tetraethyl 1,1,1,3,5,7,9,11,11,11-decamethylhexasiloxane; tetradecamethylhexasiloxane;

f) hexadecamethylheptasiloxane (L7);

g) octadecamethyloctasiloxane (L8).

Among the silicones of formula (III), mention may be made of:

a) the following tetrasiloxanes (L4):

2-[3,3,3-trimethyl-1,1-bis[(trimethylsylil)oxy]disiloxanyl]ethyl;
1,1,1,5,5,5-hexamethyl 3-(2-methylpropyl) 3-[(trimethylsilyl)oxy]trisiloxane;
3-(1,1-dimethylethyl)-1,1,1,5,5,5-hexamethyl 3-[(trimethylsilyl)oxy]trisiloxane;
3-butyl-1,1,1,5,5,5-hexamethyl-3-[(trimethylsilyl)oxy]trisiloxane;
1,1,1,5,5,5-hexamethyl-3-propyl-3-[(trimethylsilyl)oxy]trisiloxane;
3-ethyl-1,1,1,5,5,5-hexamethyl-3-[(trimethylsilyl)oxy]trisiloxane;
1,1,1-triethyl 3,5,5,5-tetramethyl-3-(trimethylsiloxy)trisiloxane;
3-methyl-1,1,1,5,5,5-hexamethyl-3-[(trimethylsilyl)oxy]trisiloxane;
3-[(dimethylphenylsilyl)oxy] 1,1,1,3,5,5,5-heptamethyltrisiloxane;
1,1,1,5,5,5-hexamethyl 3-(2-methylpentyl) 3-[(trimethylsilyl)oxy]trisiloxane;
1,1,1,5,5,5-hexamethyl 3-(4-methylpentyl) -3-[(trimethylsilyl)oxy]trisiloxane;
3-hexyl-1,1,1,5,5,5-hexamethyl-3-[(trimethylsilyl)oxy]trisiloxane;
1,1,1,3,5,5,5-heptamethyl-3-[(trimethylsilyl)oxy]trisiloxane;

b) the following pentasiloxanes (L5):

1,1,1,3,5,5,7,7,7-nonamethyl-3-(trimethylsiloxy)tetrasiloxane;
1,1,1,3,3,7,7,7-octamethyl-5-phenyl-5-[(trimethylsilyl)oxy]tetrasiloxane;

c) the following heptasiloxanes (L7):
1,1,1,3,5,5,7,7,9,9,11,11,11-tridecamethyl-3- [(trimethylsilyl)oxy]hexasiloxane.

Among the silicones of formula (IV), mention may be made of:
1,1,1,5,5,5-hexamethyl-3,3-bis(trimethylsiloxy)trisiloxane.

Use may also be made of other volatile silicone oils chosen from:

a) the following tetrasiloxanes (L4):

2,2,8,8-tetramethyl-5-[(pentamethyldisiloxanyl)methyl]- 3,7-dioxa-2,8-disilanonane;
2,2,5,8,8-pentamethyl-5-[(trimethylsilyl)methoxy] 4,6-dioxa-2,5,8-trisilanonane;
1,3-dimethyl-1,3-bis[(trimethylsilyl)methyl]-1,3-disiloxanediol;
3-ethyl-1,1,1,5,5,5-hexamethyl 3-[3-(trimethylsiloxy)propyl]trisiloxane;
1,1,1,5,5,5-hexamethyl-3-phenyl-3-[(trimethylsilyl)oxy]trisiloxane (Dow 556 Fluid);

b) the following pentasiloxanes (L5):

2,2,7,7,9,9,11,11,16,16-decamethyl-3,8,10,15-tetraoxa-2,7,9,11,16-pentasilaheptadecane;
tetrakis[(trimethylsilyl)methyl] silicic acid ester;

c) the following hexasiloxanes (L6):

3,5-diethyl 1,1,1,7,7,7-hexamethyl 3,5- bis[(trimethylsilyl)oxy]tetrasiloxane;
1,1,1,3,5,7,7,7-octamethyl-3,5-bis[(trimethylsilyl)oxy]tetrasiloxane;

d) heptasiloxane (L7):
1,1,1,3,7,7,7-heptamethyl-3,5,5-tris[(trimethylsilyl)oxy]tetrasiloxane;

e) the following octasiloxanes (L8):

1,1,1,3,5,5,9,9,9-nonamethyl-3,7,7-tris[(trimethylsilyl)oxy]pentasiloxane;
1,1,1,3,5,7,9,9,9-nonamethyl3,5,7-tris[(trimethylsilyl)oxy]pentasiloxane;
1,1,1,7,7,7-hexamethyl-3,3,5,5- tetrakis[(trimethylsilyl)oxy]tetrasiloxane.

Preferably, the non-cyclic volatile silicone oils according to the invention have a viscosity at 25°C ranging from 0.5 to 8 cSt (from 0.5 to 8 $mm^2/s$). The viscosity measurement method used in the invention to characterize the silicone oils according to the invention may be the "kinematic viscosity at 25°C raw product CID-012-01" or the "Ubbelohde viscosity at 25°C DIN 51562-1 PV04001".

Among the non-cyclic volatile silicone oils according to the invention, linear non-cyclic volatile silicone oils will more particularly be selected, and more particularly:

- octamethyltrisiloxane, in particular sold under the name Xiameter PMX-200 Silicone Fluid 1CS by Dow Corning;
- decamethyltetrasiloxane, in particular sold under the name Xiameter PMX-200 Silicone Fluid 1.5CS® by Dow Corning;
- dodecamethylpentasiloxane such as the commercial products sold under the names KF-96L-2CS®, DM-Fluid-2CS® by Shin-Etsu BERB- DM2® by BRB International or Silicone Fluid 2CS® by Dow Corning;
- mixtures thereof, and more particularly dodecamethylpentasiloxane.

Non-volatile oils

The non-volatile oils can be chosen in particular from non-volatile hydrocarbon-based, fluorinated and/or silicone oils.

Mention may in particular be made, as non-volatile hydrocarbon-based oil, of:

- hydrocarbon-based oils of plant origin, such as phytostearyl esters, such as phytostearyl oleate, phytostearyl isostearate and lauroyl/octyldodecyl/phytostearyl glutamate (Ajinomoto, Eldew PS203); triglycerides formed from fatty acid esters of glycerol, in particular whose fatty acids may have chain lengths ranging from $C_4$ to $C_{36}$, and in particular from $C_{18}$ to $C_{36}$, these oils possibly being linear or branched, and saturated or unsaturated; these oils may in particular be heptanoic or octanoic triglycerides, shea oil, alfalfa oil, poppy oil, pumpkin oil, millet oil, barley oil, quinoa oil, rye oil, candlenut oil, passionflower oil, shea butter oil, aloe oil, sweet almond oil, peach stone oil, groundnut oil, argan oil, avocado oil, baobab oil, borage oil, broccoli oil, calendula oil, camellina oil, canola oil, carrot oil, safflower oil, hemp oil, rapeseed oil, cottonseed oil, coconut oil, marrow seed oil, wheatgerm oil, jojoba oil, lily oil, macadamia oil, corn oil, meadowfoam oil, St-John's wort oil, monoi oil, hazelnut oil, apricot kernel oil, walnut oil, olive oil, evening primrose oil, palm oil, blackcurrant pip oil, kiwi seed oil, grape seed oil, pistachio oil, squash oil, pumpkin oil, musk rose oil, sesame oil, soybean oil, sunflower oil, castor oil and watermelon oil, and mixtures thereof, or alternatively caprylic/capric acid triglycerides, such as those sold by the company Stearineries Dubois or those sold under the names Miglyol 810®, 812® and 818® by the company Dynamit Nobel;

linear or branched hydrocarbons, of mineral or synthetic origin, such as paraffin oils and derivatives thereof, petroleum jelly, polydecenes, polybutenes, hydrogenated polyisobutene such as Parleam, or squalane;

synthetic ethers containing from 10 to 40 carbon atoms, such as dicaprylyl ether;

synthetic esters, such as oils of formula $R^1COOR^2$, in which $R^1$ represents a residue of a linear or branched fatty acid comprising from 1 to 40 carbon atoms, and $R^2$ represents a hydrocarbon-based chain, which is in particular branched, containing from 1 to 40 carbon atoms on condition that the sum of the number of carbon atoms of the chains $R^1$ and $R^2$ is greater than or equal to 10; the esters may be chosen in particular from fatty acid esters of alcohol, for instance cetostearyl octanoate, isopropyl alcohol esters, such as isopropyl myristate, isopropyl palmitate, ethyl palmitate, 2-ethylhexyl palmitate, isopropyl stearate, isopropyl isostearate, isostearyl isostearate, octyl stearate, hydroxylated esters, for instance isostearyl lactate, octyl hydroxystearate, diisopropyl adipate, heptanoates, and in particular isostearyl heptanoate, alcohol or polyalcohol octanoates, decanoates or ricinoleates, for instance propylene glycol dioctanoate, cetyl octanoate, tridecyl octanoate, 2-ethylhexyl 4-diheptanoate, 2-ethylhexyl palmitate, alkyl benzoate, polyethylene glycol diheptanoate, propylene glycol 2-diethylhexanoate, and mixtures thereof, $C_{12}$-$C_{15}$ alcohol benzoates, hexyl laurate, neopentanoic acid esters, for instance isodecyl neopentanoate, isotridecyl neopentanoate, isostearyl neopentanoate, octyldodecyl neopentanoate, isononanoic acid esters, for instance isononyl isononanoate, isotridecyl isononanoate, octyl isononanoate, hydroxylated esters, for instance isostearyl lactate and diisostearyl malate;

polyol esters and pentaerythritol esters, such as dipentaerythritol tetra hydroxystearate/tetraisostearate;

esters of diol dimers and of diacid dimers, such as Lusplan DD-DA5® and Lusplan DD-DA7® sold by the company Nippon Fine Chemical and described in application US 2004-175 338;
copolymers of a diol dimer and of a diacid dimer and esters thereof, such as dilinoleyl diol dimer/dilinoleic dimer copolymers and esters thereof, for instance Plandool-G;
copolymers of polyols and of diacid dimers, and esters thereof, such as Hailuscent ISDA or the dilinoleic acid/butanediol copolymer;
fatty alcohols that are liquid at ambient temperature, with a branched and/or unsaturated carbon-based chain containing from 12 to 26 carbon atoms, for instance 2-octyldodecanol, isostearyl alcohol, oleyl alcohol, 2-hexyldecanol, 2-butyloctanol and 2-undecylpentadecanol;
higher $C_{12}$-$C_{22}$ fatty acids, such as oleic acid, linoleic acid or linolenic acid, and mixtures thereof;

- dialkyl carbonates, the two alkyl chains possibly being identical or different, such as the dicaprylyl carbonate sold under the name Cetiol CC® by Cognis;
oils of high molar mass, in particular having a molar mass ranging from about 400 to about 10 000 g/mol, in particular from about 650 to about 10 000 g/mol, in particular from about 750 to about 7,500 g/mol and more particularly ranging from about 1,000 to about 5,000 g/mol, As oils of high molar mass that may be used in the present invention, mention may be made in particular of oils chosen from:

lipophilic polymers,
linear fatty acid esters with a total carbon number ranging from 35 to 70, hydroxylated esters,
aromatic esters,
$C_{24}$-$C_{28}$ branched fatty acid or fatty alcohol esters,
silicone oils,
oils of plant origin,
and mixtures thereof;
fluorinated oils which are optionally partially hydrocarbon-based and/or silicone-based, for instance fluorosilicone oils, fluorinated polyethers or fluorinated silicones, such as described in the document EP-A-847 752;
silicone oils, for instance linear or cyclic non-volatile polydimethylsiloxanes (PDMSs); polydimethylsiloxanes comprising alkyl, alkoxy or phenyl groups, which are pendant or at the end of a silicone chain, these groups containing from 2 to 24 carbon atoms, such as caprylyl methicone; phenyl silicones, for instance phenyl trimethicones, phenyl dimethicones, phenyltrimethylsiloxydiphenylsiloxanes, diphenyl dimethicones, diphenylmethyldiphenyltrisiloxanes and 2-phenylethyltrimethylsiloxysilicates, and
mixtures thereof.

Among the linear or branched hydrocarbons of mineral or synthetic origin, use is preferably made of paraffin oils or liquid petroleum jelly.
Among the hydrocarbon-based oils of plant origin, mention may preferably be made of plant oils, such as sweet almond oil, jojoba oil or macadamia oil.
According to one particular form, the hydrocarbon-based oils may be made up of liquid organic UVB-screening agents.
The liposoluble liquid organic UVB-screening agents that can be used according to the invention are preferably chosen from

- liquid lipophilic β,β-diphenylacrylate compounds
- liquid lipophilic salicylate compounds
- liquid lipophilic cinnamate compounds
- and mixtures thereof.

**β,β-Diphenylacrylate compounds**

Among these compounds, the following compounds are more particularly preferred: 2-ethylhexyl α-cyano-β,β-diphenylacrylate,

ethyl a-cyano-β,β-diphenylacrylate such as Etocrylene, sold in particular under the trade name Uvinul N35® by BASF,
2-ethylhexyl β,β-diphenylacrylate,
ethyl β,β-di(4'-methoxyphenyl)acrylate.

Among these compounds, preference is given even more particularly to the compound 2-ethylhexyl 2-cyano-3,3-diphenylacrylate, or Octocrylene, sold in particular under the trade name Uvinul N539 by BASF.

**Salicylate compounds**

Among the liquid lipophilic salicylate compounds that can be used according to the invention, mention may be made of:

- Homosalate sold under the name Eusolex HMS by Rona/EM Industries,
- Ethylhexyl salicylate, sold under the name Neo Heliopan OS by Symrise.

**Cinnamate compounds**

Among the liquid lipophilic cinnamate compounds that can be used according to the invention, mention may be made of:

- Ethylhexyl Methoxycinnamate, sold in particular under the trade name Parsol MCX® by DSM Nutritional Products,
- Isopropyl Methoxycinnamate,
- Isoamyl Methoxycinnamate, sold under the trade name Neo Heliopan E 1000 by Symrise.

Among the liquid liposoluble UVB-screening agents according to the invention, use will preferably be made of the compounds chosen from:

- octocrylene,
- homosalate,
- ethylhexyl salicylate,
- ethylhexyl methoxycinnamate,

and mixtures thereof.

Among the liquid liposoluble UVB-screening agents, use will more preferentially be made of the compounds chosen from:

- octocrylene,
- ethylhexyl salicylate,
- homosalate,

and mixtures thereof.

According to one preferential embodiment of the invention, the mixture of β,β-diphenylacrylate / salicylate screening agents will be present in the composition of the invention at a concentration of at least 15% by weight, and more preferentially at a concentration of at least 20% by weight relative to the total weight of the composition.

Preferably, said mixture of β,β-diphenylacrylate / salicylate screening agents is used in concentrations ranging from 15% to 40% by weight, and more preferentially from 20% to 30% by weight relative to the total weight of the composition.

**ADDITIVES**

The compositions according to the invention can in addition comprise additives commonly used in care and/or makeup products, such as

- active agents such as vitamins, for example vitamins A, E, C, $B_3$; baicalin or plant extracts containing same, in particular of root of Skullcap and in particular Scutellaria baicalensis (INCI name: Scutellaria baicalensis root extract); moisturizing agents;
- sunscreens;
- pigments;
- additional fillers different from the organopolysiloxane elastomer powders coated with silicone resin and lipophilic clays;
- water-soluble colorants and/or liposoluble colorants;
- and mixtures thereof.

It is a matter of routine operation for those skilled in the art to adjust the nature and the amount of the additives present in the compositions in accordance with the invention such that the desired cosmetic properties thereof are not thereby affected.

**UV-screening agents**

The compositions according to the invention may also contain one or more UV-screening agents chosen from organic UV-screening agents and/or inorganic screening agents.

i) Organic UV-screening agents

The organic UV-screening agents are in particular chosen from cinnamic compounds; diphenylacrylate compounds; salicylate compounds; dibenzoylmethane compounds; anthranilate compounds; benzylidenecamphor compounds; benzophenone compounds; triazine compounds; benzotriazole compounds, in particular the silicone benzotriazoles described in patent EP0392883 and the methylenebis(hydroxyphenyl benzotriazole) compounds as described in applications US 5 237 071, US 5 166 355, GB2303549, DE 197 26 184 and EP893119; benzalmalonate compounds, in particular those mentioned in patent US 5 624 663; benzimidazole compounds; imidazoline compounds; the bis-benzoazolyl compounds as described in patents EP669323 and US 2 463 264; the benzoxazole compounds as described in patent applications EP0832642, EP1027883, EP1300137 and DE10162844; screening polymers and screening silicones such as those described in particular in application WO-93/04665; merocyanine compounds as described in US 4 195 999, application WO2004/006878, applications WO2008/090066, WO2011113718, WO2009027258, WO2013010590, WO2013011094, WO2013011480 and the documents IP COM JOURNAL N°000179675D published on 23 February 2009, IP COM JOURNAL N°000182396D published on 29 April 2009, IP COM JOURNAL N° 000189542D published on 12 November 2009, and IP COM Journal N°IPCOM000011179D published on 04/03/2004, and mixtures thereof.

As examples of organic photoprotective agents, mention may be made of those denoted hereinbelow under their INCI name:

Dibenzoylmethane compounds

Butyl methoxydibenzoylmethane sold in particular under the trade name Parsol 1789® by DSM Nutritional Products, Inc.;

Cinnamic compounds:

Ethylhexyl methoxycinnamate, sold in particular under the trade name Parsol MCX® by DSM Nutritional Products,
Isopropyl methoxycinnamate,
Isoamyl p-methoxycinnamate sold under the trade name Neo Heliopan E 1000® by Symrise,

Salicylic compounds:

Homosalate, sold under the name Eusolex HMS® by Rona/EM Industries,
Ethylhexyl salicylate, sold under the name Neo Heliopan OS® by Symrise,
Dipropylene glycol salicylate, sold under the name Dipsal® by Scher,
TEA salicylate, sold under the name Neo Heliopan TS® by Symrise,

β,β-Diphenylacrylate compounds:

Octocrylene, sold in particular under the trade name Uvinul N 539® by BASF, Etocrylene, sold in particular under the trade name Uvinul N 35® by BASF.

Benzophenone compounds:

Benzophenone-1 sold under the trade name Uvinul 400® by BASF,
Benzophenone-2, sold under the trade name Uvinul D 50® by BASF,
Benzophenone-3 or Oxybenzone, sold under the trade name Uvinul M 40® by BASF,
Benzophenone-4, sold under the trade name Uvinul MS 40® by BASF,
Benzophenone-5,
Benzophenone-6 sold under the trade name Helisorb 11® by Norquay,
Benzophenone-8, sold under the trade name Spectra-Sorb UV-24® by American Cyanamid,

Benzophenone-9, sold under the trade name Uvinul DS 49® by BASF,
Benzophenone-12,
n-Hexyl 2-(4-diethylamino-2-hydroxybenzoyl)benzoate, sold under the trade name Uvinul A Plus® or, as a mixture with octyl methoxycinnamate, under the trade name Uvinul A Plus B® by the company BASF,
1,1'-(1,4-Piperazinediyl)bis[1-[2-[4-(diethylamino)-2-hydroxybenzoyl]phenyl]methanone] (CAS 919803-06-8), as described in application WO 2007/071 584; this compound advantageously being used in micronized form (mean size of 0.02 to 2 μm), which may be obtained, for example, according to the micronization process described in applications GB-A-2 303 549 and EP-A-893 119, and in particular in the form of an aqueous dispersion.

Benzylidenecamphor compounds:

3-Benzylidenecamphor, manufactured under the name Mexoryl SD® by Chimex, 4-Methylbenzylidenecamphor, sold under the name Eusolex 6300® by Merck, Benzylidenecamphorsulfonic acid, manufactured under the name Mexoryl SL® by Chimex,
Camphor benzalkonium methosulfate, manufactured under the name Mexoryl SO® by Chimex,
Terephthalylidenedicamphorsulfonic acid, manufactured under the name Mexoryl SX® by Chimex,
Polyacrylamidomethylbenzylidenecamphor, manufactured under the name Mexoryl SW® by Chimex.

Phenylbenzimidazole compounds:

Phenylbenzimidazolesulfonic acid, sold in particular under the trade name Eusolex 232® by Merck.

Bis-benzoazolyl compounds:

Disodium phenyl dibenzimidazole tetrasulfonate, sold under the trade name Neo Heliopan AP® by Symrise.

Benzotriazole compounds

Drometrizole trisiloxane, manufactured under the name Mexoryl SX® by Chimex;

Methylene bis-Benzotriazolyl Tetramethylbutylphenol in particular in solid form, such as the product sold under the trade name MIXXIM BB/100® by Fairmount Chemical or in the form of an aqueous dispersion of micronized particles having a mean particle size which ranges from 0.01 to 5 μm and more preferentially from 0.01 to 2 μm and more particularly from 0.020 to 2 μm, with at least one alkylpolyglycoside surfactant of structure:
$CnH_{2n+1}$ $O(C_6H_{10}O_5)_xH$ in which n is an integer from 8 to 16 and x is the average degree of polymerization of the $(C_6H_{10}O_5)$ unit and ranges from 1.4 to 1.6, as described in patent GB-A-2 303 549, in particular sold under the trade name Tinosorb M® by the company BASF or in the form of an aqueous dispersion of micronized particles having a mean particle size which ranges from 0.02 to 2 μm and more preferentially from 0.01 to 1.5 μm and more particularly from 0.02 to 1 μm in the presence of at least one mono-$(C_8-C_{20})$alkyl ester of polyglycerol having a degree of glycerol polymerization of at least 5, such as the aqueous dispersions described in application WO2009/063392.

Triazine compounds:

- Bis-Ethylhexyloxyphenol methoxyphenyl triazine, sold under the trade name Tinosorb S® by BASF,
- Ethylhexyl triazone, sold in particular under the trade name Uvinul T150® by BASF,
- Diethylhexyl butamido triazone, sold under the trade name Uvasorb HEB® by Sigma 3V,
- symmetrical triazine screening agents substituted with naphthalenyl groups or polyphenyl groups described in patent US 6 225 467, application WO 2004/085 412 (see compounds 6 and 9) or the document "Symmetrical Triazine Derivatives", IP.COM IPCOM000031257 Journal, INC, West Henrietta, NY, US (20 September 2004), in particular 2,4,6-tris(diphenyl)triazine and 2,4,6-tris(terphenyl)triazine, which is also mentioned in patent applications WO 06/035 000, WO 06/034 982, WO 06/034 991, WO 06/035 007, WO 2006/034 992 and WO 2006/034 985, these compounds advantageously being used in micronized form (mean particle size of 0.02 to 3 μm), which may be obtained, for example, according to the micronization process described in applications GB-A-2 303 549 and EP-A-893 119, and in particular in aqueous dispersion;

- silicone triazines substituted with two aminobenzoate groups, as described in patent EP 0 841 341, in particular 2,4-bis(n-butyl 4'-aminobenzalmalonate)-6-[(3-{1,3,3,3-tetramethyl-1-[(trimethylsilyl)oxy]disiloxanyl}propyl)amino]-s-triazine.

Anthranilic compounds:

Menthyl anthranilate, sold under the trade name Neo Heliopan MA® by Symrise.

Imidazoline compounds:

Ethylhexyl dimethoxybenzylidene dioxoimidazoline propionate,

Benzalmalonate compounds:

Polyorganosiloxane containing benzalmalonate functional groups, for instance Polysilicone-15, sold under the trade name Parsol SLX® by DSM Nutritional Products, Inc.

Benzoxazole compounds:

2,4-bis[5-(1,1-dimethylpropyl)benzoxazol-2-yl(4-phenyl)imino]-6-(2-ethylhexyl)imino-1,3,5-triazine, sold under the name Uvasorb K2A® by Sigma 3V.

ii) Inorganic UV screening agents

The inorganic UV-screening agents used in accordance with the present invention are metal oxide pigments. More preferentially, the inorganic UV-screening agents of the invention are metal oxide particles with a mean elementary particle size of less than or equal to 0.5 μm, more preferentially between 0.005 and 0.5 μm, even more preferentially between 0.01 and 0.2 μm, better still between 0.01 and 0.1 μm and more particularly between 0.015 and 0.05 μm.

They may be chosen in particular from titanium oxide, zinc oxide, iron oxide, zirconium oxide and cerium oxide, or mixtures thereof.

Such coated or uncoated metal oxide pigments are described in particular in patent application EP-A-0 518 773. Commercial pigments that may be mentioned include the products sold by the companies Sachtleben Pigments, Tayca, Merck and Degussa.

The metal oxide pigments may be coated or uncoated.

The coated pigments are pigments that have undergone one or more surface treatments of chemical, electronic, mechanochemical and/or mechanical nature with compounds such as amino acids, beeswax, fatty acids, fatty alcohols, anionic surfactants, lecithins, sodium, potassium, zinc, iron or aluminium salts of fatty acids, metal alkoxides (of titanium or aluminium), polyethylene, silicones, proteins (collagen, elastin), alkanolamines, silicon oxides, metal oxides or sodium hexametaphosphate.

The coated pigments are more particularly titanium oxides that have been coated:

- with silica, such as the product Sunveil® from the company Ikeda,
- with silica and iron oxide, such as the product Sunveil F® from the company Ikeda,
- with silica and alumina, such as the products Microtitanium Dioxide MT 500 SA® and Microtitanium Dioxide MT 100 SA from the company Tayca and Tioveil from the company Tioxide,
- with alumina, such as the products Tipaque TTO-55 (B)® and Tipaque TTO-55 (A)® from the company Ishihara and UVT 14/4 from the company Sachtleben Pigments,
- with alumina and aluminium stearate, such as the products Microtitanium Dioxide MT 100 T®, MT 100 TX®, MT 100 Z® and MT-01® from the company Tayca, the products Solaveil CT-10 W® and Solaveil CT 100® from the company Uniqema and the product Eusolex T-AVO® from the company Merck,
- with silica, alumina and alginic acid, such as the product MT-100 AQ® from the company Tayca,
- with alumina and aluminium laurate, such as the product Microtitanium Dioxide MT 100 S® from the company Tayca,
- with iron oxide and iron stearate, such as the product Microtitanium Dioxide MT 100 F® from the company Tayca,
- with zinc oxide and zinc stearate, such as the product BR 351® from the company Tayca,
- with silica and alumina and treated with a silicone, such as the products Microtitanium Dioxide MT 600 SAS®, Microtitanium Dioxide MT 500 SAS® or Microtitanium Dioxide MT 100 SAS® from the company Tayca,
- with silica, alumina and aluminium stearate and treated with a silicone, such as the product STT-30-DS® from the

company Titan Kogyo,
- with silica and treated with a silicone, such as the product UV-Titan X 195® from the company Sachtleben Pigments,
- with alumina and treated with a silicone, such as the products Tipaque TTO-55 (S)® from the company Ishihara or UV Titan M 262® from the company Sachtleben Pigments,
- with triethanolamine, such as the product STT-65-S from the company Titan Kogyo,
- with stearic acid, such as the product Tipaque TTO-55 (C)® from the company Ishihara,
- with sodium hexametaphosphate, such as the product Microtitanium Dioxide MT 150 W® from the company Tayca,
- $TiO_2$ treated with octyltrimethylsilane, sold under the trade name T 805® by the company Degussa Silices,
- $TiO_2$ treated with a polydimethylsiloxane, sold under the trade name 70250 Cardre UF TiO2SI3® by the company Cardre,
- anatase/rutile $TiO_2$ treated with a polydimethylhydrogenosiloxane, sold under the trade name Micro Titanium Dioxide USP Grade Hydrophobic® by the company Color Techniques.

Mention may also be made of $TiO_2$ pigments doped with at least one transition metal such as iron, zinc or manganese and more particularly manganese. Preferably, said doped pigments are in the form of an oily dispersion. The oil present in the oily dispersion is preferably chosen from triglycerides including those of capric/caprylic acids. The oily dispersion of titanium oxide particles may also comprise one or more dispersants, for instance a sorbitan ester, for instance sorbitan isostearate, or a polyoxyalkylenated fatty acid ester of glycerol, for instance TRI-PPG-3 myristyl ether citrate and polyglyceryl-3 polyricinoleate. Preferably, the oily dispersion of titanium oxide particles comprises at least one dispersant chosen from polyoxyalkylenated fatty acid esters of glycerol. Mention may be made more particularly of the oily dispersion of $TiO_2$ particles doped with manganese in capric/caprylic acid triglyceride in the presence of TRI-PPG-3 myristyl ether citrate and polyglyceryl-3 polyricinoleate and sorbitan isostearate having the INCI name: titanium dioxide (and) TRI-PPG-3 myristyl ether citrate (and) polyglyceryl-3 ricinoleate (and) sorbitan isostearate, for instance the product sold under the trade name Optisol TD50® by the company Croda.

The uncoated titanium oxide pigments are sold, for example, by the company Tayca under the trade names Microtitanium Dioxide MT 500 B or Microtitanium Dioxide MT 600 B®, by the company Degussa under the name P 25, by the company Wackher under the name Transparent titanium oxide PW®, by the company Miyoshi Kasei under the name LIFTR®, by the company Tomen under the name ITS® and by the company Tioxide under the name Tioveil AQ.

The uncoated zinc oxide pigments are for example:

- those sold under the name Z-Cote by Sunsmart;
- those sold under the name Nanox® by the company Elementis;
- those sold under the name Nanogard WCD 2025® by the company Nanophase Technologies.

The coated zinc oxide pigments are for example:

- those sold under the name Zinc Oxide CS-5® by the company Toshibi (ZnO coated with polymethylhydrogenosiloxane);
- those sold under the name Nanogard Zinc Oxide FN® by the company Nanophase Technologies (as a 40% dispersion in Finsolv TN®, $C_{12}$-$C_{15}$ alkyl benzoate);
- those sold under the name Daitopersion Zn-30® and Daitopersion Zn-50® by the company Daito (dispersions in cyclopolymethylsiloxane/oxyethylenated polydimethylsiloxane, containing 30% or 50% of zinc oxides coated with silica and polymethylhydrogenosiloxane);
- those sold under the name NFD Ultrafine ZnO® by the company Daikin (ZnO coated with perfluoroalkyl phosphate and copolymer based on perfluoroalkylethyl as a dispersion in cyclopentasiloxane);
- those sold under the name SPD-Z1® by the company Shin-Etsu (ZnO coated with silicone-grafted acrylic polymer, dispersed in cyclodimethylsiloxane);
- those sold under the name Escalol Z100® by the company ISP (alumina-treated ZnO dispersed in the ethylhexyl methoxycinnamate/PVP-hexadecene copolymer/methicone mixture);
- those sold under the name Fuji ZnO-SMS-10® by the company Fuji Pigment (ZnO coated with silica and polymethylsilsesquioxane);
- those sold under the name Nanox Gel TN® by the company Elementis (ZnO dispersed at a concentration of 55% in $C_{12}$-$C_{15}$ alkyl benzoate with hydroxystearic acid polycondensate).

The uncoated cerium oxide pigments may be, for example, those sold under the name Colloidal Cerium Oxide® by the company Rhone-Poulenc.

The uncoated iron oxide pigments are sold, for example, by the company Arnaud under the names Nanogard WCD 2002® (FE 45B®), Nanogard Iron FE 45 BL AQ, Nanogard FE 45R AQ® and Nanogard WCD 2006® (FE 45R®)

or by the company Mitsubishi under the name TY-220®.

The coated iron oxide pigments are sold, for example, by the company Arnaud under the names Nanogard WCD 2008 (FE 45B FN)®, Nanogard WCD 2009® (FE 45B 556®), Nanogard FE 45 BL 345® and Nanogard FE 45 BL® or by the company BASF under the name Transparent Iron Oxide®.

Mention may also be made of mixtures of metal oxides, in particular of titanium dioxide and of cerium dioxide, including the equal-weight mixture of titanium dioxide and cerium dioxide coated with silica, sold by the company Ikeda under the name Sunveil A®, and also the mixture of titanium dioxide and zinc dioxide coated with alumina, silica and silicone, such as the product M 261® sold by the company Sachtleben Pigments, or coated with alumina, silica and glycerol, such as the product M 211® sold by the company Sachtleben Pigments.

According to the invention, the coated or uncoated inorganic screening agents based on titanium oxide are particularly preferred.

The UV-screening agents according to the invention are preferably present in the compositions according to the invention in a content ranging from 0.1% to 45% by weight and in particular from 5% to 30% by weight relative to the total weight of the composition.

## Pigments

According to one particular embodiment of the invention, the composition according to the invention comprises at least one pigment.

The term "pigments" is intended to mean white or coloured, mineral or organic particles, which are insoluble in an aqueous medium, and which are intended to colour and/or opacify the resulting composition and/or deposit. These pigments may be white or coloured, and mineral and/or organic.

Preferably, the composition comprises at least 5% by weight of pigments, more preferentially from 5% to 40% by weight of pigments, in particular from 10% to 30% by weight and preferably from 10% to 20% by weight of pigments, relative to the total weight of said composition.

According to a particular embodiment, the pigments used according to the invention are chosen from mineral pigments.

The term "mineral pigment" is intended to mean any pigment that satisfies the definition in Ullmann's encyclopaedia in the chapter on inorganic pigments. Among the mineral pigments that are useful in the present invention, mention may be made of zirconium oxide or cerium oxide, and also zinc oxide, iron oxide (black, yellow or red) or chromium oxide, manganese violet, ultramarine blue, chromium hydrate and ferric blue, titanium dioxide, and metal powders, for instance aluminium powder and copper powder. The following mineral pigments may also be used: $Ta_2O_5$, $Ti_3O_5$, $Ti_2O_3$, TiO, $ZrO_2$ as a mixture with $TiO_2$, $ZrO_2$, $Nb_2O_5$, $CeO_2$, ZnS.

The size of the pigment that is useful in the context of the present invention is generally greater than 100 nm and can range up to 10 μm , preferably from 200 nm to 5 μm and more preferentially from 300 nm to 1 μm.

According to one particular embodiment of the invention, the pigments have a size characterized by a D[50] greater than 100 nm and possibly ranging up to 10 μm, preferably from 200 nm to 5 μm and more preferentially from 300 nm to 1 μm.

The sizes are measured by static light scattering using a commercial MasterSizer 3000 particle size analyzer from Malvern, which makes it possible to determine the particle size distribution of all of the particles over a wide range which may extend from 0.01 μm to 1000 μm. The data are processed on the basis of the standard Mie scattering theory. This theory is the one most suitable for size distributions ranging from submicron to multimicron; it allows an "effective" particle diameter to be determined. This theory is described in particular in the publication by Van de Hulst, H.C., Light Scattering by Small Particles, Chapters 9 and 10, Wiley, New York, 1957.

D[50] represents the maximum size that 50% by volume of the particles have.

In the context of the present invention, the mineral pigments are more particularly iron oxide and/or titanium dioxide. Examples that may be mentioned more particularly include titanium dioxides and iron oxide coated with aluminium stearoyl glutamate, sold, for example, under the reference NAI by the company Miyoshi Kasei.

Mention may also be made, as inorganic pigments which can be used in the invention, of pearlescent agents.

The term "pearlescent agents" should be understood as meaning coloured particles of any shape, which are or are not iridescent, in particular produced by certain molluscs in their shells or else synthesized, and which exhibit a colour effect via optical interference.

The pearlescent agents may be chosen from nacreous pigments such as titanium mica coated with an iron oxide, titanium mica coated with bismuth oxychloride, titanium mica coated with chromium oxide, titanium mica coated with an organic dye and also nacreous pigments based on bismuth oxychloride. They may also be mica particles, at the surface of which are superposed at least two successive layers of metal oxides and/or of organic colorants.

Examples of nacres that may also be mentioned include natural mica covered with titanium oxide, with iron oxide, with natural pigment or with bismuth oxychloride.

Among the nacres available on the market, mention may be made of the nacres Timica, Flamenco and Duochrome (based on mica) sold by the company Engelhard, the Timiron nacres sold by the company Merck, the Prestige mica-based nacres sold by the company Eckart, and the Sunshine synthetic mica-based nacres sold by the company Sun Chemical.

The nacres may more particularly have a yellow, pink, red, bronze, orangey, brown, gold and/or coppery colour or glint.

Mention may in particular be made, by way of illustration of pearlescent agents which can be used in the context of the present invention, of pearlescent agents of gold colour sold in particular by Engelhard under the names Brilliant Gold 212G (Timica), Gold 222C (Cloisonne), Sparkle Gold (Timica), Gold 4504 (Chromalite) and Monarch Gold 233X (Cloisonne); bronze pearlescent agents sold in particular by Merck under the names Bronze Fine (17384) (Colorona) and Bronze (17353) (Colorona) and by Engelhard under the name Super Bronze (Cloisonne); orange pearlescent agents sold in particular by Engelhard under the names Orange 363C (Cloisonne) and Orange MCR 101 (Cosmica) and by Merck under the names Passion Orange (Colorona) and Matte Orange (17449) (Microna); brown-coloured pearlescent agents sold in particular by Engelhard under the names Nu-Antique Copper 340XB (Cloisonne) and Brown CL4509 (Chromalite); pearlescent agents with a copper glint sold in particular by Engelhard under the name Copper 340A (Timica); pearlescent agents with a red glint sold in particular by Merck under the name Sienna Fine (17386) (Colorona); pearlescent agents with a yellow glint sold in particular by Engelhard under the name Yellow (4502) (Chromalite); red-coloured pearlescent agents with a gold glint sold in particular by Engelhard under the name Sunstone G012 (Gemtone); pink pearlescent agents sold in particular by Engelhard under the name Tan Opale G005 (Gemtone); black pearlescent agents with a gold glint sold in particular by Engelhard under the name Nu-Antique Bronze 240 AB (Timica); blue pearlescent agents sold in particular by Merck under the name Matte Blue (17433) (Microna); white pearlescent agents with a silvery glint sold in particular by Merck under the name Xirona Silver; and golden green pinkish orangey pearlescent agents sold in particular by Merck under the name Indian Summer (Xirona), and mixtures thereof.

Mention may also be made, among the pigments which can be used according to the invention, of those having an optical effect different from a simple conventional colouring effect, that is to say a unified and stabilized effect such as produced by conventional colorants, such as, for example, monochromatic pigments. Within the meaning of the invention, the term "stabilized" is intended to mean devoid of an effect of variability in the colour with the angle of observation or alternatively in response to a change in temperature.

For example, this material can be chosen from particles with a metallic glint, goniochromatic colouring agents, diffracting pigments, thermochromic agents, optical brighteners and also fibres, in particular interference fibres. Of course, these various materials can be combined so as to provide the simultaneous display of two effects, indeed even of a novel effect in accordance with the invention.

The particles with a metallic glint that may be used in the invention are in particular chosen from:

- particles of at least one metal and/or of at least one metal derivative,
- particles comprising a monomaterial or multimaterial organic or mineral substrate, at least partially coated with at least one layer with a metallic glint comprising at least one metal and/or at least one metal derivative; and
- mixtures of said particles.

Among the metals that may be present in said particles, mention may be made, for example, of Ag, Au, Cu, Al, Ni, Sn, Mg, Cr, Mo, Ti, Zr, Pt, Va, Rb, W, Zn, Ge, Te and Se, and mixtures or alloys thereof. Ag, Au, Cu, Al, Zn, Ni, Mo, Cr and mixtures thereof or alloys (for example, bronzes and brasses) are preferred metals.

The term "metal derivatives" denotes compounds derived from metals, in particular oxides, fluorides, chlorides and sulfides.

Mention may be made, by way of illustration of these particles, of aluminium particles, such as those sold under the names Starbrite 1200 EAC® by Silberline and Metalure® by Eckart.

Mention may also be made of metal powders formed of copper or alloy mixtures, such as the references 2844 sold by Radium Bronze, metal pigments, such as aluminium or bronze, for example those sold under the names Rotosafe 700 from Eckart, silica-coated aluminium particles sold under the name Visionaire Bright Silver from Eckart and particles formed of metal alloy, such as powders formed of bronze (copper and zinc alloy) coated with silica sold under the name Visionaire Bright Natural Gold from Eckart.

The particles can also comprise a glass substrate, such as those sold by Nippon Sheet Glass under the names Microglass Metashine.

The goniochromatic colouring agent can be chosen, for example, from interference multilayer structures and liquid crystal colouring agents.

Examples of symmetrical interference multilayer structures which can be used in compositions produced in accordance with the invention are, for example, the following structures: $Al/SiO_2/Al/SiO_2/Al$, pigments having this structure being sold by the company DuPont de Nemours; $Cr/MgF_2/Al/MgF_2/Cr$, pigments having this structure being sold under

the name Chromaflair by the company Flex; $MoS_2/SiO_2/Al/SiO_2/MoS_2$; $Fe_2O_3/SiO_2/Al/SiO_2/Fe_2O_3$, and $Fe_2O_3/SiO_2/Fe_2O_3/SiO_2/Fe_2O_3$, pigments having these structures being sold under the name Sicopearl by the company BASF; $MoS_2/SiO_2$/mica-oxide/$SiO_2/MoS_2$; $Fe_2O_3/SiO_2$/mica-oxide/$SiO_2/Fe_2O_3$; $TiO_2/SiO_2/TiO_2$ and $TiO_2/Al_2O_3/TiO_2$; $SnO/TiO_2/SiO_2/TiO_2/SnO$; $Fe_2O_3/SiO_2/Fe_2O_3$; $SnO$/mica/$TiO_2/SiO_2/TiO_2$/mica/$SnO$, pigments having these structures being sold under the name Xirona by the company Merck (Darmstadt). By way of example, these pigments can be pigments with a silica/titanium oxide/tin oxide structure sold under the name Xirona Magic by Merck, pigments with a silica/brown iron oxide structure sold under the name Xirona Indian Summer by Merck and pigments with a silica/titanium oxide/mica/tin oxide structure sold under the name Xirona Caribbean Blue by Merck. Mention may also be made of the Infinite Colors pigments from Shiseido. Different effects are obtained according to the thickness and the nature of the various layers. Thus, with the $Fe_2O_3/SiO_2/Al/SiO_2/Fe_2O_3$ structure, the colour changes from greenish gold to reddish grey for $SiO_2$ layers of 320 to 350 nm; from red to gold for $SiO_2$ layers of 380 to 400 nm; from violet to green for $SiO_2$ layers of 410 to 420 nm; from copper to red for $SiO_2$ layers of 430 to 440 nm.

Mention may be made, as examples of pigments with a polymeric multilayer structure, of those sold by 3M under the name Color Glitter.

Use may be made, as liquid crystal goniochromatic particles, for example, of those sold by Chenix and of that sold under the name Helicone® HC by Wacker.

Hydrophobic coated pigments

Preferably, the compositions according to the invention comprise at least one pigment coated with at least one lipophilic or hydrophobic compound and in particular as detailed below.

This type of pigment is particularly advantageous insofar as it may be considered in a large amount together with a large amount of water. What is more, insofar as they are treated with a hydrophobic compound, they show a predominant affinity for the oily gelled phase, which can then convey them.

Needless to say, the compositions according to the invention may in parallel contain uncoated pigments.

The coating may also comprise at least one additional non-lipophilic compound. For the purposes of the invention, the "coating" of a pigment according to the invention generally denotes the total or partial surface treatment of the pigment with a surface agent, absorbed, adsorbed or grafted onto said pigment.

The surface-treated pigments may be prepared according to surface treatment techniques of chemical, electronic, mechanochemical or mechanical nature that are well known to those skilled in the art. Commercial products may also be used.

The surface agent may be absorbed, adsorbed or grafted onto the pigments by evaporation of solvent, chemical reaction and creation of a covalent bond.

According to one variant, the surface treatment is constituted of a coating of the pigments.

The coating may represent from 0.1% to 20% by weight and in particular from 0.5% to 5% by weight, relative to the total weight of the coated pigment.

The coating may be performed, for example, by adsorption of a liquid surface agent onto the surface of the solid particles by simple mixing with stirring of the particles and of said surface agent, optionally with heating, prior to the incorporation of the particles into the other ingredients of the makeup or care composition.

The coating may be performed, for example, by chemical reaction of a surface agent with the surface of the solid pigment particles and creation of a covalent bond between the surface agent and the particles. This method is in particular described in patent US 4 578 266.

The chemical surface treatment may consist in diluting the surface agent in a volatile solvent, dispersing the pigments in this mixture and then slowly evaporating off the volatile solvent, so that the surface agent is deposited at the surface of the pigments.

Lipophilic or hydrophobic treatment agent

When the pigment comprises a lipophilic or hydrophobic coating, it is preferably present in the fatty phase of the composition according to the invention.

According to a particular embodiment of the invention, the pigments may be coated according to the invention with at least one compound chosen from silicone surface agents; fluoro surface agents; fluorosilicone surface agents; metal soaps; N-acylamino acids or salts thereof; lecithin and derivatives thereof; isopropyl triisostearyl titanate; isostearyl sebacate; natural plant or animal waxes; polar synthetic waxes; fatty esters; phospholipids; and mixtures thereof.

Silicone surface agent

According to a particular embodiment, the pigments may be totally or partially surface-treated with a compound

of silicone nature.

The silicone surface agents may be chosen from organopolysiloxanes, silane derivatives, silicone-acrylate copolymers, silicone resins, and mixtures thereof.

The term "organo-polysiloxane compound" is intended to mean a compound having a structure comprising an alternance of silicone atoms and oxygen atoms and comprising organic radicals linked to silicon atoms.

Non-elastomeric organopolysiloxane

Non-elastomeric organopolysiloxanes that may in particular be mentioned include polydimethylsiloxanes, polymethylhydrogenosiloxanes and polyalkoxydimethylsiloxanes.

The alkoxy group may be represented by the radical R-O- such that R represents methyl, ethyl, propyl, butyl or octyl, 2-phenylethyl, 2-phenylpropyl or 3,3,3-trifluoropropyl radicals, aryl radicals such as phenyl, tolyl or xylyl, or substituted aryl radicals such as phenylethyl.

One method for surface-treating pigments with a polymethylhydrogenosiloxane consists in dispersing the pigments in an organic solvent and then in adding the silicone compound. On heating the mixture, covalent bonds are created between the silicone compound and the surface of the pigment.

According to one preferred embodiment, the silicone surface agent may be a non-elastomeric organopolysiloxane, in particular chosen from polydimethylsiloxanes.

Alkylsilanes and alkoxysilanes

Silanes containing alkoxy functionality are in particular described by Witucki in "A silane primer, chemistry and applications of alkoxy silanes", Journal of Coatings Technology, 65, 822, pages 57-60, 1993.

Alkoxysilanes such as the alkyltriethoxysilanes and the alkyltrimethoxysilanes sold under the references Silquest A-137 (OSI Specialities) and Prosil 9202 (PCR) may be used for coating the pigments.

The use of alkylpolysiloxanes bearing a reactive end group such as alkoxy, hydroxyl, halogen, amino or imino is described in application JP H07-196946. They are also suitable for treating the pigments.

Silicone-acrylate polymers

Grafted silicone-acrylic polymers having a silicone backbone as described in patents US 5 725 882, US 5 209 924, US 4 972 037, US 4 981 903, US 4 981 902 and US 5 468 477 and in patents US 5 219 560 and EP 0 388 582 may be used.

Other silicone-acrylate polymers may be silicone polymers comprising in their structure the unit of formula (I) below:

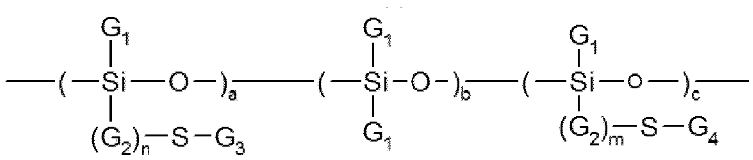

(I)

in which the radicals $G_1$, which may be identical or different, represent hydrogen or a $C_1$-$C_{10}$ alkyl radical or alternatively a phenyl radical; the radicals $G_2$, which may be identical or different, represent a $C_1$-$C_{10}$ alkylene group; $G_3$ represents a polymeric residue resulting from the (homo)polymerization of at least one ethylenically unsaturated anionic monomer; $G_4$ represents a polymeric residue resulting from the (homo)polymerization of at least one ethylenically unsaturated hydrophobic monomer; m and n are equal to 0 or 1; a is an integer ranging from 0 to 50; b is an integer that may be between 10 and 350, c is an integer ranging from 0 to 50; with the proviso that one of the parameters a and c is other than 0.

Preferably, the unit of formula (I) above has at least one, and even more preferentially all, of the following characteristics:

- the radicals $G_1$ denote an alkyl radical, preferably a methyl radical;
- n is non-zero, and the radicals $G_2$ represent a divalent $C_1$-$C_3$ radical, preferably a propylene radical;
- $G_3$ represents a polymeric radical resulting from the (homo)polymerization of at least one monomer of the ethylenically unsaturated carboxylic acid type, preferably acrylic acid and/or methacrylic acid;
- $G_4$ represents a polymeric radical resulting from the (homo)polymerization of at least one monomer of the ($C_1$-$C_{10}$)alkyl (meth)acrylate type, preferably such as isobutyl or methyl (meth)acrylate.

Examples of silicone polymers corresponding to formula (I) are in particular polydimethylsiloxanes (PDMS) onto which are grafted, via a connecting chain unit of thiopropylene type, mixed polymer units of the poly(meth)acrylic acid type and of the polymethyl (meth)acrylate type.

Other examples of silicone polymers corresponding to formula (I) are in particular polydimethylsiloxanes (PDMS) onto which are grafted, via a connecting chain unit of thiopropylene type, polymer units of the polyisobutyl (meth)acrylate type.

Silicone resins

The silicone surface agent may be chosen from silicone resins.

The term "resin" is intended to mean a three-dimensional structure.

The silicone resins may be soluble or swellable in silicone oils. These resins are crosslinked polyorganosiloxane polymers.

The nomenclature of silicone resins is known under the name "MDTQ", the resin being described as a function of the various siloxane monomer units that it comprises, each of the letters "MDTQ" characterizing a type of unit.

The letter M represents the monofunctional unit of formula $(CH_3)_3SiO_{1/2}$, the silicon atom being bonded to only one oxygen atom in the polymer comprising this unit.

The letter D means a difunctional unit $(CH_3)_2SiO_{2/2}$ in which the silicon atom is bonded to two oxygen atoms.

The letter T represents a trifunctional unit of formula $(CH_3)SiO_{3/2}$.

In the units M, D and T defined above, at least one of the methyl groups may be substituted with a group R other than a methyl group, such as a hydrocarbon-based radical (in particular alkyl) containing from 2 to 10 carbon atoms or a phenyl group, or alternatively a hydroxyl group.

Finally, the letter Q means a tetrafunctional unit $SiO_{4/2}$ in which the silicon atom is bonded to four hydrogen atoms, which are themselves bonded to the rest of the polymer.

Various resins with different properties may be obtained from these different units, the properties of these polymers varying as a function of the type of monomers (or units), of the type and number of substituted radicals, of the length of the polymer chain, of the degree of branching and of the size of the side chains.

Examples of these silicone resins that may be mentioned include:

- siloxysilicates, which may be trimethyl siloxysilicates of formula $[(CH_3)_3XSiXO]_xX(SiO_{4/2})_y$ (MQ units) in which x and y are integers ranging from 50 to 80;
- polysilsesquioxanes of formula $(CH_3SiO_{3/2})_x$ (T units) in which x is greater than 100 and at least one of the methyl radicals of which may be substituted with a group R as defined above;
- polymethylsilsesquioxanes, which are polysilsesquioxanes in which none of the methyl radicals is substituted with another group.

Such polymethylsilsesquioxanes are described in document US 5 246 694.

As examples of commercially available polymethylsilsesquioxane resins, mention may be made of those sold:

- by the company Wacker under the reference Resin MK, such as Belsil PMS MK: polymer comprising $CH_3SiO_{3/2}$ repeating units (T units), which may also comprise up to 1% by weight of $(CH_3)_2SiO_{2/2}$ units (D units) and having an average molecular weight of about 10 000, or
- by the company Shin-Etsu under the references KR-220L, which are composed of units T of formula $CH_3SiO_{3/2}$ and contain Si-OH (silanol) end groups, under the reference KR-242A, which comprise 98% of units T and 2% of dimethyl units D and contain Si-OH end groups, or else under the reference KR-251, comprising 88% of units T and 12% of dimethyl units D and contain Si-OH end groups.

Siloxysilicate resins that may be mentioned include trimethyl siloxysilicate (TMS) resins, optionally in the form of powders. Such resins are sold under the references SR1000, E 1 170-002 or SS 4230, by the company General Electric or under the references TMS 803, Wacker 803 and 804 by the company Wacker Silicone Corporation.

Mention may also be made of trimethylsiloxysilicate resins sold in a solvent such as cyclomethicone, sold under the name KF-7312J by the company Shin-Etsu or DC 749 and DC 593 by the company Dow Corning.

As examples of commercial references of pigments treated with a silicone compound, mention may be made of:

- red iron oxide/dimethicone sold under the reference SA-C 338075-10 by the company Miyoshi Kasei, and
- a pigment obtained by treating DC Red 7 with a silicone compound, sold by the company Coletica under the reference Gransil GCM (which is a mixture of D5 and polysilicone 11).

Fluoro surface agent

The pigments may be totally or partially surface-treated with a compound of fluoro nature.
The fluoro surface agents may be chosen from perfluoroalkyl phosphates, perfluoropolyethers, polytetrafluoropolyethylenes (PTFE), perfluoroalkanes, perfluoroalkyl silazanes, polyhexafluoropropylene oxides, and polyorganosiloxanes comprising perfluoroalkyl perfluoropolyether groups.
The term "perfluoroalkyl radical" is intended to mean an alkyl radical in which all of the hydrogen atoms have been replaced with fluorine atoms.
Perfluoropolyethers are in particular described in patent application EP 0 486 135, and sold under the trade name Fomblin by the company Montefluos.
Perfluoroalkyl phosphates are in particular described in application JP H05-86984. The perfluoroalkyl diethanolamine phosphates sold by Asahi Glass under the reference AsahiGuard AG530 may be used.
Among the linear perfluoroalkanes that may be mentioned are perfluorocycloalkanes, perfluoro(alkylcycloalkanes), perfluoropolycycloalkanes, aromatic perfluoro hydrocarbons (perfluoroarenes) and hydrocarbon-based perfluoro organic compounds comprising at least one heteroatom.
Among the perfluoroalkanes, mention may be made of the linear alkane series such as perfluorooctane, perfluorononane or perfluorodecane.
Among the perfluorocycloalkanes and perfluoro-(alkylcycloalkanes), mention may be made of perfluorodecalin sold under the name Flutec PP5 GMP by the company Rhodia, perfluoro(methyldecalin) and perfluoro($C_3$-$C_5$ alkylcyclohexanes) such as perfluoro-(butylcyclohexane).
Among the perfluoropolycycloalkanes, mention may be made of bicyclo[3.3.1]nonane derivatives such as perfluorotrimethylbicyclo[3.3.1]nonane, adamantane derivatives such as perfluorodimethyladamantane, and hydrogenated perfluorophenanthrene derivatives such as tetracosafluorotetradecahydrophenanthrene.
Among the perfluoroarenes, mention may be made of perfluoronaphthalene derivatives, for instance perfluoronaphthalene and perfluoromethyl-1-naphthalene.
As examples of commercial references of pigments treated with a fluoro compound, mention may be made of:

- yellow iron oxide/perfluoroalkyl phosphate sold under the reference PF 5 Yellow 601 by the company Daito Kasei;
- red iron oxide/perfluoroalkyl phosphate sold under the reference PF 5 Red R 516L by the company Daito Kasei;
- black iron oxide/perfluoroalkyl phosphate sold under the reference PF 5 Black BL 100 by the company Daito Kasei;
- titanium dioxide/perfluoroalkyl phosphate sold under the reference PF 5 $TiO_2$ CR 50 by the company Daito Kasei,
- yellow iron oxide/perfluoropolymethyl isopropyl ether sold under the reference Iron oxide yellow BF-25-3 by the company Toshiki;
- DC Red 7/perfluoropolymethyl isopropyl ether sold under the reference D&C Red 7 FHC by the company Cardre Inc.; and
- DC Red 6/PTFE sold under the reference T 9506 by the company Warner-Jenkinson.

Fluorosilicone surface agent

The pigments may be totally or partially surface-treated with a compound of fluorosilicone nature.
The fluorosilicone compound may be chosen from perfluoroalkyl dimethicones, perfluoroalkyl silanes and perfluoroalkyl trialkoxysilanes.
Perfluoroalkyl silanes that may be mentioned include the products LP-IT and LP-4T sold by Shin-Etsu Silicone.
The perfluoroalkyl dimethicones may be represented by the following formula:

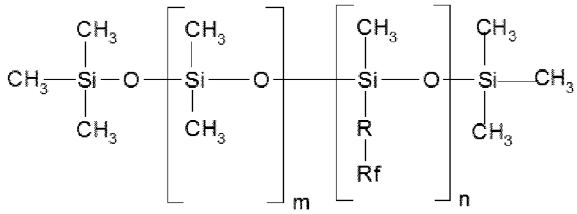

in which:

- R represents a linear or branched divalent alkyl group containing from 1 to 6 carbon atoms, preferably a divalent methyl, ethyl, propyl or butyl group;
- Rf represents a perfluoroalkyl radical containing 1 to 9 carbon atoms and preferably 1 to 4 carbon atoms;

- m is chosen between 0 and 150 and preferably from 20 to 100; and
- n is chosen between 1 and 300 and preferably from 1 to 100.

As examples of commercial references of pigments treated with a fluorosilicone compound, mention may be made of titanium dioxide/fluorosilicone sold under the reference Fluorosil Titanium dioxide 100TA by the company Advanced Dermaceuticals International Inc.

Other lipophilic surface agents

The hydrophobic treatment agent may also be chosen from:

i) metal soaps such as aluminium dimyristate and the aluminium salt of hydrogenated tallow glutamate;

Metal soaps that may in particular be mentioned include metal soaps of fatty acids containing from 12 to 22 carbon atoms and in particular those containing from 12 to 18 carbon atoms.
The metal of the metal soap may in particular be zinc or magnesium.
Metal soaps that may be used include zinc laurate, magnesium stearate, magnesium myristate and zinc stearate, and mixtures thereof.

ii) fatty acids such as lauric acid, myristic acid, stearic acid and palmitic acid;
iii) N-acylamino acids or salts thereof, which may comprise an acyl group containing from 8 to 22 carbon atoms, for instance a 2-ethylhexanoyl, caproyl, lauroyl, myristoyl, palmitoyl, stearoyl or cocoyl group.

The amino acid may be, for example, lysine, glutamic acid or alanine.
The salts of these compounds can be the aluminium, magnesium, calcium, zirconium, zinc, sodium or potassium salts.
Thus, according to a particularly preferred embodiment, an N-acylamino acid derivative may in particular be a glutamic acid derivative and/or a salt thereof, and more particularly a stearoyl glutamate, for instance aluminium stearoyl glutamate.

iv) lecithin and derivatives thereof;
v) isopropyl triisostearyl titanate;

As examples of isopropyl titanium triisostearate (ITT)-treated pigments, mention may be made of those sold under the commercial references BWBO-I2 (Iron oxide CI77499 and isopropyl titanium triisostearate), BWYO-I2 (Iron oxide CI77492 and isopropyl titanium triisostearate) and BWRO-O2 (Iron oxide CI77491 and isopropyl titanium triisostearate) by the company Kobo.

vi) isostearyl sebacate;
vii) natural plant or animal waxes or polar synthetic waxes;
viii) fatty esters, in particular jojoba esters;
ix) phospholipids; and
x) mixtures thereof.

The waxes mentioned in the compounds mentioned previously may be those generally used in cosmetics, as defined hereinbelow.
They may in particular be hydrocarbon-based, silicone and/or fluoro waxes, optionally comprising ester or hydroxyl functions. They may also be of natural or synthetic origin.
The term "polar wax" is intended to mean a wax containing chemical compounds comprising at least one polar group. Polar groups are well known to those skilled in the art; they may be, for example, alcohol, ester or carboxylic acid groups. Polyethylene waxes, paraffin waxes, microcrystalline waxes, ozokerite and Fischer-Tropsch waxes are not included among polar waxes.
In particular, the polar waxes have a mean Hansen solubility parameter δa at 25°C such that $\delta a > 0\ (J/cm^3)^{1/2}$ and better still $\delta a > 1\ (J/cm^3)^{1/2}$: $\delta_a = \sqrt{\delta_p^2 + \delta_h^2}$ in which δp and δh are, respectively, the polar contributions and contributions of interaction types specific to the Hansen solubility parameters.
The definition of solvents in the three-dimensional solubility space according to Hansen is described in the

article by C. M. Hansen: "The three-dimensional solubility parameters", J. Paint Technol. 39, 105 (1967):

- δh characterizes the specific interaction forces (such as hydrogen bonding, acid/base, donor/acceptor, etc.);
- δp characterizes the Debye interaction forces between permanent dipoles and also the Keesom interaction forces between induced dipoles and permanent dipoles.

The parameters δp and δh are expressed in 1 $(J/cm^3)^{1/2}$.
A polar wax is in particular formed from molecules comprising, besides carbon and hydrogen atoms in their chemical structure, heteroatoms (such as O, N and P).
Non-limiting illustrations of these polar waxes that may in particular be mentioned include natural polar waxes, such as beeswax, lanolin wax, orange wax, lemon wax and Chinese insect waxes, rice bran wax, carnauba wax, candelilla wax, ouricury wax, cork fibre wax, sugarcane wax, Japan wax, sumac wax and montan wax.
According to a particular embodiment, the pigments may be coated with at least one compound chosen from silicone surface agents; fluoro surface agents; N-acylamino acids or salts thereof; isopropyl triisostearyl titanate; natural plant or animal waxes; fatty esters; and mixtures thereof.
According to a particularly preferred embodiment, the pigments may be coated with an N-acylamino acid and/or a salt thereof, in particular with a glutamic acid derivative and/or a salt thereof, or with a fatty ester, in particular with a jojoba ester.
According to a more particularly preferred embodiment, the pigments may be coated with an N-acylamino acid and/or a salt thereof, in particular with a glutamic acid derivative and/or a salt thereof, in particular a stearoyl glutamate, for instance aluminium stearoyl glutamate.
Examples of coated pigments according to the invention that may be mentioned more particularly include titanium dioxides and iron oxide coated with aluminium stearoyl glutamate, sold, for example, under the reference NAI by Miyoshi Kasei.

Pigments not coated with a hydrophobic compound

As stated previously, a composition may also contain pigments not coated with a lipophilic or hydrophobic compound.
These other pigments may be coated with a hydrophilic compound or uncoated.
These pigments may be mineral pigments in particular as defined previously.
These pigments may also be organic pigments.
The term "organic pigment" is intended to mean any pigment that satisfies the definition in Ullmann's encyclopaedia in the chapter on organic pigments. The organic pigment may in particular be chosen from nitroso, nitro, azo, xanthene, quinoline, anthraquinone, phthalocyanine, metal complex, isoindolinone, isoindoline, quinacridone, perinone, perylene, diketopyrrolopyrrole, thioindigo, dioxazine, triphenylmethane and quinophthalone compounds.
The organic pigment(s) may be chosen, for example, from carmine, carbon black, aniline black, melanin, azo yellow, quinacridone, phthalocyanine blue, sorghum red, the blue pigments codified in the Color Index under the references CI 42090, 69800, 69825, 73000, 74100 and 74160, the yellow pigments codified in the Color Index under the references CI 11680, 11710, 15985, 19140, 20040, 21100, 21108, 47000 and 47005, the green pigments codified in the Color Index under the references CI 61565, 61570 and 74260, the orange pigments codified in the Color Index under the references CI 11725, 15510, 45370 and 71105, the red pigments codified in the Color Index under the references CI 12085, 12120, 12370, 12420, 12490, 14700, 15525, 15580, 15620, 15630, 15800, 15850, 15865, 15880, 17200, 26100, 45380, 45410, 58000, 73360, 73915 and 75470, and the pigments obtained by oxidative polymerization of indole or phenolic derivatives as described in patent FR 2 679 771.
These pigments may also be in the form of composite pigments as described in patent EP 1 184 426. These composite pigments may in particular be composed of particles comprising an inorganic core at least partially covered with an organic pigment and at least one binder for fixing the organic pigments to the core.
The pigment may also be a lake. The term "lake" is intended to mean insolubilized dyes adsorbed onto insoluble particles, the assembly thus obtained remaining insoluble during use.
The inorganic substrates onto which the dyes are adsorbed are, for example, alumina, silica, calcium sodium borosilicate or calcium aluminium borosilicate, and aluminium.
Among the organic dyes, mention may be made of cochineal carmine. Mention may also be made of the products known under the following names: D&C Red 21 (CI 45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), D&C Yellow 5 (CI 19 140), D&C Yellow 6 (CI 15 985), D&C Green (CI 61 570), D&C Yellow 1 O (CI 77 002), D&C Green 3 (CI 42 053), D&C Blue 1 (CI 42 090).
By way of examples of lakes, mention may be made of the product known under the name D&C Red 7 (CI 15

850:1).

Nature of the hydrophilic coating

As stated previously, these other pigments may be coated with a hydrophilic compound.
Said hydrophilic compound for surface-treating a pigment in order to optimize its dispersion in the gelled aqueous phase is more particularly chosen from biological polymers, carbohydrates, polysaccharides, polyacrylates and polyethylene glycol derivatives.
As examples of biological polymers, mention may be made of polymers based on monomers of carbohydrate type.
More particularly, mention may be made of biosaccharide gum, chitosans and derivatives thereof, such as butoxy chitosan, carboxymethyl chitosan, carboxybutyl chitosan, chitosan gluconate, chitosan adipate, chitosan glycolate, chitosan lactate, etc., chitins and derivatives thereof, such as carboxymethyl chitin, chitin glycolate; cellulose and derivatives thereof such as cellulose acetate; microcrystalline cellulose; distarch phosphate; sodium hyaluronate; soluble proteoglycans; galacto-arabinans; glycosaminoglycans; glycogen; sclerotium gum; dextran; starch and derivatives thereof; and mixtures thereof.
Examples of carbohydrates that may in particular be mentioned include polyhydroxyaldehydes or polyhydroxy ketones of general formula: Cx(H2O)y in which x and y may range from 1 to 1 000 000.
The carbohydrates may be monosaccharides, disaccharides or polysaccharides.
Examples of carbohydrates that may in particular be mentioned include amylodextrins, beta-glucans, cyclodextrins, modified corn starch, glycogen, hyaluronic acid, hydroxypropylcyclodextrin, lactose, maltitol, guanosine, glyceryl starch, *Triticum vulgare* starch, trehalose, sucrose and derivatives thereof, raffinose and sodium chondroitin sulfate.
$C_1$-$C_{20}$ alkylene glycols or $C_1$-$C_{20}$ alkylene glycol ethers, alone or used in combination with tri($C_1$-$C_{20}$)alkylsilanes, may also be used as surface-treatment agents.
Examples that may be mentioned include pigments surface-treated with PEG alkyl ether alkoxysilane, for instance pigments treated with PEG-8-methyl ether triethoxysilane sold by the company Kobo under the name SW pigments.
Silicones such as dimethicones bearing hydrophilic groups, also known under the name dimethicone copolyols or alkyl dimethicone copolyols, may also be suitable for use in the invention as surface treatment agents. In particular, such dimethicones may comprise, as repeating units, $C_1$-$C_{20}$ alkylene oxides, such as ethylene or propylene oxides.
An example that may be mentioned is the pigment treated with PEG-12-dimethicone, sold by the company Sensient Corporation under the name LCW AQ Pigment.
The amount of pigments coated with at least one hydrophilic compound and/or of uncoated pigments is in particular conditioned by the intended use of the cosmetic composition under consideration, and the adjustment of this amount obviously falls within the competence of the composition formulator.

**Additional fillers**

The fillers which can be used in the compositions of the invention can be of organic or inorganic nature and make it possible in particular to confer on it additional properties of improved stability, wear property, coverage and/or mattness.
The content of filler(s) can range from 2% to 20% by weight, in particular from 4% to 12% by weight, with respect to the total weight of said composition.
The fillers used in the compositions according to the present invention can be of lamellar, globular, spherical or fibrous forms or of any other form intermediate between these defined forms.
The fillers according to the invention may or may not be surface-coated, and in particular they may be surface-treated with silicones, amino acids, fluorinated derivatives or any other substance which promotes the dispersion and the compatibility of the filler in the composition.
Mention may be made, as examples of inorganic fillers, of clays, talc, mica, silica, hollow silica microspheres, kaolin, calcium carbonate, magnesium carbonate, hydroxyapatite, boron nitride, glass or ceramic microcapsules, composites of silica and of titanium dioxide, such as the TSG series sold by Nippon Sheet Glass, or hydrophobic silica aerogel particles surface-modified by trimethylsilyl groups.
According to a specific form of the invention, the composition of the invention comprises, as filler, at least hydrophobic silica aerogel particles surface-modified by trimethylsilyl groups and/or a lipophilic clay.
As hydrophobic silica aerogels that may be used in the invention, examples that may be mentioned include the aerogel sold under the name VM-2260® (INCI name: Silica silylate), by the company Dow Corning, the particles of which have an average size of about 1000 microns and a specific surface area per unit mean of mass ranging from 600 to 800 $m^2/g$.

Mention may also be made of the aerogels sold by Cabot under the references Aerogel TLD 201, Aerogel OGD 201, Aerogel TLD 203, Enova® Aerogel MT 1100 and Enova Aerogel MT 1200.

Use will preferably be made of the aerogel sold under the name VM-2270 (INCI name: Silica silylate) by Dow Corning, the particles of which have a mean size ranging from 5-15 microns and a specific surface area per unit of mass ranging from 600 to 800 $m^2/g$.

Mention may be made, as examples of organic fillers, of powders formed of polyamide (Orgasol Nylon® from Atochem), of polyethylene, of poly(methyl methacrylate), of polytetrafluoroethylene (Teflon) or of acrylic acid copolymers (Polytrap from Dow Corning), lauroyl lysine, hollow polymeric microspheres, such as those of polyvinylidene chloride/acrylonitrile, such as Expancel (Nobel Industrie), hexamethylene diisocyanate/trimethylol hexyllactone copolymer powder (Plastic Powder from Toshiki), silicone resin microbeads (Tospearl from Toshiba, for example), synthetic or natural micronized waxes, metal soaps derived from organic carboxylic acids having from 8 to 22 carbon atoms, preferably from 12 to 18 carbon atoms, for example zinc stearate, magnesium stearate, lithium stearate, zinc laurate or magnesium myristate, Polypore® L 200 (Chemdal Corporation) or polyurethane powders, in particular powders formed of crosslinked polyurethane comprising a copolymer, said copolymer comprising trimethylol hexyllactone. It may in particular be a hexamethylene diisocyanate/trimethylol hexyllactone polymer. Such particles are in particular commercially available, for example under the name Plastic Powder D-400® or Plastic Powder D-800® from Toshiki, and mixtures thereof.

## Water-soluble or liposoluble colorants

A composition according to the invention may also comprise at least one water-soluble colorant and/or one liposoluble colorant, preferably in a proportion of at least 0.01% by weight by rapliposoluble and preferably a proportion of least 0.01% by weight relative to the total weight of the composition.

For obvious reasons, this amount is liable to vary significantly with regard to the intensity of the desired colour effect and of the colour intensity afforded by the colorants under consideration, and its adjustment clearly falls within the competence of those skilled in the art.

The additional colorants that are suitable for use in the invention may be water-soluble, but may also be liposoluble.

For the purposes of the invention, the term "water-soluble colorant" is intended to mean any natural or synthetic, generally organic compound, which is soluble in an aqueous phase or water-miscible solvents and which is capable of colouring.

As water-soluble dyes that are suitable for use in the invention, mention may be made in particular of synthetic or natural water-soluble dyes, for instance FDC Red 4, DC Red 6, DC Red 22, DC Red 28, DC Red 30, DC Red 33, DC Orange 4, DC Yellow 5, DC Yellow 6, DC Yellow 8, FDC Green 3, DC Green 5, FDC Blue 1, betanine (beetroot), carmine, copper chlorophylline, methylene blue, anthocyanins (enocianin, black carrot, hibiscus and elder), caramel and riboflavin.

The water-soluble dyes are, for example, beetroot juice and caramel.

For the purposes of the invention, the term "liposoluble colorant" is intended to mean any natural or synthetic, generally organic compound, which is soluble in an oily phase or in solvents that are miscible with a fatty substance, and which is capable of imparting colour.

As liposoluble dyes that are suitable for use in the invention, mention may be made in particular of synthetic or natural liposoluble dyes, for instance DC Red 17, DC Red 21, DC Red 27, DC Green 6, DC Yellow 11, DC Violet 2, DC Orange 5, Sudan red, carotenes (β-carotene, lycopene), xanthophylls (capsanthin, capsorubin, lutein), palm oil, Sudan brown, quinoline yellow, annatto and curcumin.

## APPLICATIONS

According to one embodiment, a composition of the invention may advantageously be in the form of a composition for caring for the skin and/or keratin fibres, the body or the face, in particular the face.

According to another embodiment, a composition of the invention can advantageously be in the form of a composition for making up keratin materials, in particular the skin of the body or of the face, in particular of the face.

Thus, according to a sub-mode of this embodiment, a composition of the invention may advantageously be in the form of a makeup base composition.

A composition of the invention may advantageously be in the form of a foundation.

According to another sub-mode of this embodiment, a composition of the invention may advantageously be in the form of a composition for making up the skin and in particular the face. It may thus be an eyeshadow or a face powder.

Such compositions are in particular prepared according to the general knowledge of those skilled in the art.

Throughout the description, including the claims, the term "comprising a" should be understood as being synonymous with "comprising at least one", unless otherwise specified.

The expressions "between ... and ...", and "ranging from ... to ..." should be understood as meaning limits

included, unless otherwise specified.

The invention is illustrated in more detail by the examples and figures presented below. Unless otherwise indicated, the amounts shown are expressed as percentages by weight.

EXAMPLE: Foundation in the form of a water/oil emulsion

| Phase | **Ingredients** | **EX 1** (invention) |
|---|---|---|
| A1 | POLYGLYCERYL-4 DIISOSTEARATE/POLYHYDROXYSTEARATE SEBACATE (ISOLAN GPS® from EVONIK) | 0.75 |
| | PEG-30 DIPOLYHYDROXYSTEARATE (CITHROL DPHS-SO-(MV) from the company CRODA | 100 |
| A2 | HOMOSALATE | 10 |
| | ETHYLHEXYL SALICYLATE | 5 |
| | OCTOCRYLENE | 7 |
| A3 | DODECAMETHYLPENTASILOXANE PDMS 2CST (SILICONE FLUID 2CS® from DOW CORNING) | 16.00 |
| A4 | DISTEARDIMONIUM HECTORITE (BENTONE V38 ® from ELEMENTIS) | 0.75 |
| A5 | VINYL DIMETHICONE/METHICONE SILSESQUIOXANE CROSSPOLYMER (KSP 100® from SHIN ETSU) | 200 |
| A6 | VITAMINE TOCOPHEROL | 100 |
| B | PROPANEDIOL | 5 |
| | SCUTELLARIA BAICALENSIS ROOT EXTRACT (BAICALIN 95 MM® from MMP) | 0.2 |
| | MAGNESIUM SULFATE | 0.7000 |
| | WATER | qs 100 |
| | PHENOXYETHANOL | 0.50 |
| | NIACINAMIDE | 2 |
| | CAFFEINE | 1 |
| C | ALCOHOL | 5 |
| D | YELLOW IRON OXIDE COATED WITH ALUMINIUM STEAROYL GLUTAMATE RED IRON OXIDE COATED WITH ALUMINIUM STEAROYL GLUTAMATE BLACK IRON OXIDE COATED WITH ALUMINIUM STEAROYL GLUTAMATE TITANIUM DIOXIDE COATED WITH ALUMINIUM STEAROYL GLUTAMATE | 14 |

**Preparation protocol**

**Preparation of the aqueous phase B:**

In a glass beaker: the Niacinamide was dissolved in water with stirring using a magnetic bar, then the caffeine was added in order to obtain a transparent solution, then the baicalin was added in order to obtain a clear yellow transparent solution with a pH of 4.7 and then the $MgSO_4$ salt, propanediol and phenoxyethanol were added in order to obtain a clear yellow transparent solution with a pH of 4.75.

**Preparation of the oily phase:**

In a capsule, a part of the silicone (two times the amount of clay) was taken in order to wet the clay with a

flexible spatula. On a hot plate, in the main beaker, the ethylhexyl salicylate was heated with the PEG-30 dipolyhydroxystearate and the polyglyceryl-4 diisostearate/polyhydroxystearate/sebacate to around 55°C, until the PEG-30 dipolyhydroxystearate had melted, and then the mixture was cooled by adding the other two sunscreens (the temperature drops to 30°C) and the rest of the silicone was added.

**Emulsification:**

The aqueous phase was added little by little, with stirring on a Moritz stirrer, to the oily phase in order to obtain a concentrated white base, the stirring speed was increased and the mixture was left to stir for 10 minutes until a fluid mixture was obtained. The mixture of modified clay and volatile silicone oil was added to the white base and the resulting mixture was stirred in a turbine very strongly in order to activate the clay, and was left to stir for 15 minutes until the mixture had thickened. The KSP 100 was added, with stirring on a Moritz stirrer, and the mixture was left for 10 minutes until it had thickened. The vitamin E and then the pigments were added until there was good development of the colour. The formula was then debubbled.

After production, the appearance of formula 1 at ambient temperature was smooth, uniform and fluid. The stability of formula 1, which is placed in a 30 ml transparent glass mortar for preparing ointment, so that it fills it to 2/3 of its volume, was evaluated:

- at ambient temperature for 2 months;
- in an incubator at 4°C for 2 months;
- in an incubator at 45°C for 2 months.

The results obtained are indicated in the following table:

| **Stability measured** | **EX 1 (invention)** |
|---|---|
| Ambient temperature for 2 months | Stable |
| 4°C for 2 months | Stable |
| 45°C for 2 months | Stable |

The composition of Example 1 is storage-stable equally at ambient temperature, 4°C and 45°C. After application, it has good sensory cosmetic properties, such as a non-tacky effect, a non-greasy effect, the absence of an unpleasant film sensation after application, and a feeling of lightness.

## Claims

1. Composition, in particular comprising a physiologically acceptable medium, in particular for coating keratin materials, more particularly for making up and/or caring for keratin materials, such as the skin, in the form of a water-in-oil emulsion comprising:

    a) a continuous oily phase, and
    b) at least one discontinuous aqueous phase dispersed in said oily phase;
    c) at least one emulsifying system comprising:

        i) at least one fatty acid ester of a polyol and
        ii) at least one polymer of polyoxyethylenated fatty acid ester of glycol; and

    d) at least one lipophilic clay; and
    e) at least one organopolysiloxane elastomer powder coated with a silicone resin.

2. Composition according to Claim 1, in which the fatty acid ester of a polyol is polyglyceryl-4 diisostearate polyhydroxystearate sebacate of formula:

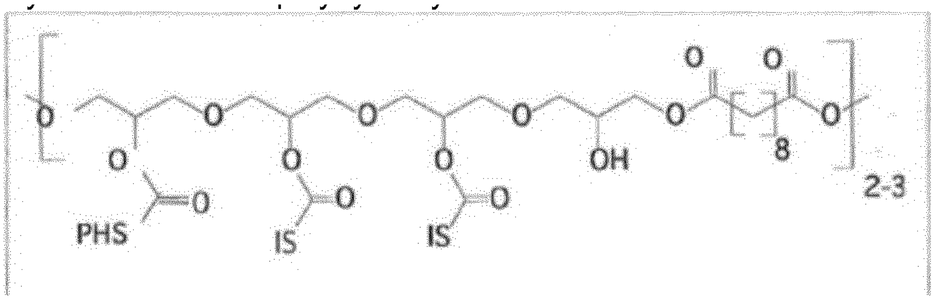

in which PHS denotes polyhydroxystearic acid and IS denotes isostearic acid, having the INCI name: Polyglyceryl-4 Diisostearate/Polyhydroxystearate/Sebacate.

**3.** Composition according to Claim 1, in which the polymer of polyoxyethylenated fatty acid ester of glycol is polyethylene glycol dipolyhydroxystearate containing 30 EO having the INCI name: PEG-30 Dipolyhydroxystearate.

**4.** Composition according to any one of the preceding claims, in which:

i) the fatty acid ester of a polyol is preferably present in the composition in contents ranging from 0.1% to 5% by weight, more preferentially from 0.1% to 2% by weight and more particularly from 0.1% to 0.75% by weight, relative to the total weight of the composition; and
ii) the polymer of polyoxyethylenated fatty acid ester of glycol is preferably present in the composition in contents ranging from 0.1% to 5% by weight, more preferentially from 0.1% to 2% by weight and more particularly from 0.1% to 1% by weight, relative to the total weight of the composition.

**5.** Composition according to any one of the preceding claims, in which the lipophilic clay is chosen from bentonites which have been hydrophobically modified and hectorites which have been hydrophobically modified, in particular with a $C_{10}$ to $C_{22}$ quaternary ammonium chloride, in particular stearalkonium chloride or distearyldimethylammonium chloride.

**6.** Composition according to any one of the preceding claims, in which the lipophilic clay(s) are present in the composition in concentrations ranging preferably from 0.1% to 5% by weight and more preferentially from 0.1% to 1% by weight relative to the total weight of the composition.

**7.** Composition according to any one of the preceding claims, in which the organopolysiloxane elastomer powder is coated with a silsesquioxane resin, and more particularly chosen from:

- those with the INCI name: Vinyl Dimethicone/Methicone Silsesquioxane Crosspolymer;
- those with the INCI name: Trifluoropropyl Dimethicone/ Vinyl Trifluoropropyldimethicone/Silsesquioxane Crosspolymer;
- those with the INCI name Diphenyl Dimethicone/Vinyl Diphenyl Dimethicone/Silsesquioxane Crosspolymer;
and mixtures thereof.

**8.** Composition according to any one of the preceding claims, in which the organopolysiloxane elastomer powder(s) coated with silicone resin may be present in a content ranging from 0.1% to 10% by weight, preferably from 0.5% to 5% by weight, more particularly from 0.5% to 2% by weight relative to the total weight of said composition.

**9.** Composition according to any one of the preceding claims, comprising at least one additive chosen from:

- active agents such as vitamins, moisturizing agents;
- sunscreens;
- pigments;
- fillers;
- water-soluble colorants and/or liposoluble colorants;
- and mixtures thereof.

**10.** Composition according to any one of the preceding claims, comprising at least one pigment, preferably coated with

at least one lipophilic or hydrophobic compound, in particular hydrophobic coated pigments of iron oxide and/or of titanium dioxide.

**11.** Composition according to any one of the preceding claims, being in the form of a foundation.

**12.** Process for coating keratin materials, more particularly for making up and/or caring for keratin materials, such as the skin, **characterized in that** it comprises the application to the keratin materials of a composition as defined according to any one of Claims 1 to 11.

## Patentansprüche

**1.** Zusammensetzung, insbesondere umfassend ein physiologisch unbedenkliches Medium, insbesondere zum Beschichten von Keratinmaterialien, insbesondere zum Schminken und/oder Pflegen von Keratinmaterialien, wie der Haut, in Form einer Wasser-in-Öl-Emulsion, umfassend:

a) eine kontinuierliche ölige Phase und
b) mindestens einen diskontinuierliche wässrige Phase, die in der öligen Phase dispergiert ist;
c) mindestens ein Emulgiersystem, umfassend:

i) mindestens einen Fettsäureester eines Polyols und
ii) mindestens ein Polymer von polyoxyethyleniertem Fettsäureester von Glykol; und

d) mindestens einen lipophilen Ton und
e) mindestens ein mit einem Silikonharz beschichtetes Organopolysiloxanelastomerpulver.

**2.** Zusammensetzung nach Anspruch 1, wobei es sich bei dem Fettsäureester eines Polyols um Polyglyceryl-4-diisostearatpolyhydroxystearatsebacat der Formel:

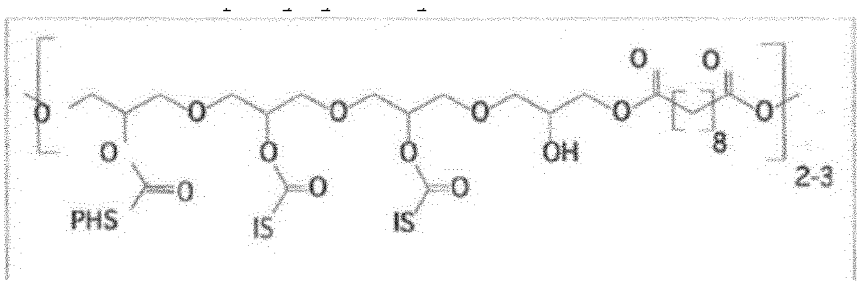

wobei PHS für Polyhydroxystearinsäure steht und IS für Isostearinsäure steht, mit dem INCI-Namen Polyglyceryl-4 Diisostearate/Polyhydroxystearate/Sebacate handelt.

**3.** Zusammensetzung nach Anspruch 1, wobei es sich bei dem Polymer von polyoxyethyleniertem Fettsäureester von Glykol um Polyethylenglykoldipolyhydroxystearat mit 30 EO mit dem INCI-Namen PEG-30 Dipolyhydroxystearate handelt.

**4.** Zusammensetzung nach einem der vorhergehenden Ansprüche, wobei:

i) der Fettsäureester eines Polyols vorzugsweise in der Zusammensetzung in Gehalten im Bereich von 0,1 bis 5 Gew.-%, weiter bevorzugt von 0,1 bis 2 Gew.-% und spezieller von 0,1 bis 0,75 Gew.-%, bezogen auf das Gesamtgewicht der Zusammensetzung, vorliegt und
ii) das Polymer von polyoxyethyleniertem Fettsäureester von Glykol vorzugsweise in der Zusammensetzung in Gehalten im Bereich von 0,1 bis 5 Gew.-%, weiter bevorzugt von 0,1 bis 2 Gew.-% und spezieller von 0,1 bis 1 Gew.-%, bezogen auf das Gesamtgewicht der Zusammensetzung, vorliegt.

**5.** Zusammensetzung nach einem der vorhergehenden Ansprüche, wobei der lipophilen Ton ausgewählt ist aus Bentoniten, die hydrophob modifiziert wurden, und Hectoriten, die hydrophob modifiziert wurden, insbesondere mit einem quartären $C_{10}$- bis $C_{22}$-Ammoniumchlorid, insbesondere Stearalkoniumchlorid oder Distearyldimethylammoniumchlorid.

6. Zusammensetzung nach einem der vorhergehenden Ansprüche, wobei der lipophile Ton bzw. die lipophilen Tone in der Zusammensetzung in Konzentrationen im Bereich von vorzugsweise 0,1 bis 5 Gew.-% und weiter bevorzugt von 0,1 bis 1 Gew.-%, bezogen auf das Gesamtgewicht der Zusammensetzung, vorliegt bzw. vorliegen.

7. Zusammensetzung nach einem der vorhergehenden Ansprüche, wobei das Organopolysiloxanelastomerpulver mit einem Silsesquioxanharz beschichtet ist, das spezieller ausgewählt ist aus:

   - denjenigen mit dem INCI-Namen Vinyl Dimethicone/Methicone Silsesquioxane Crosspolymer;
   - denjenigen mit dem INCI-Namen Trifluoropropyl Dimethicone/Vinyl Trifluoropropyldimethicone/Silsesquioxane Crosspolymer;
   - denjenigen mit dem INCI-Namen Diphenyl Dimethicone/Vinyl Diphenyl Dimethicone/ Silsesquioxane Crosspolymer und Mischungen davon.

8. Zusammensetzung nach einem der vorhergehenden Ansprüche, wobei das mit Silikonharz beschichtete Organopolysiloxanelastomerpulver bzw. die mit Silikonharz beschichteten Organopolysiloxanelastomerpulver in einem Gehalt im Bereich von 0,1 bis 10 Gew.-%, vorzugsweise von 0,5 bis 5 Gew.-%, spezieller von 0,5 bis 2 Gew.-%, bezogen auf das Gesamtgewicht der Zusammensetzung, vorliegen kann bzw. können.

9. Zusammensetzung nach einem der vorhergehenden Ansprüche, umfassend mindestens ein Additiv, ausgewählt aus:

   - Wirkstoffen wie Vitaminen, feuchtigkeitsspendenden Mitteln;
   - Lichtschutzmitteln;
   - Pigmenten;
   - Füllstoffen;
   - wasserlöslichen Farbmitteln und/oder fettlöslichen Farbmitteln
   - und Mischungen davon.

10. Zusammensetzung nach einem der vorhergehenden Ansprüche, umfassend mindestens ein Pigment, das vorzugsweise mit mindestens einer lipophilen oder hydrophoben Verbindung beschichtet ist, insbesondere hydrophob beschichtete Pigmente von Eisenoxid und/oder Titandioxid.

11. Zusammensetzung nach einem der vorhergehenden Ansprüche, die in Form einer Foundation vorliegt.

12. Verfahren zum Beschichten von Keratinmaterialien, insbesondere zum Schminken und/oder Pflegen von Keratinmaterialien, wie der Haut, **dadurch gekennzeichnet, dass** es das Aufbringen einer Zusammensetzung gemäß einem der Ansprüche 1 bis 11 auf die Keratinmaterialien umfasst.

## Revendications

1. Composition, en particulier comprenant un milieu physiologiquement acceptable, en particulier pour le revêtement de matériaux kératiniques, plus particulièrement pour le maquillage et/ou le soin de matériaux kératiniques, tels que la peau, sous la forme d'une émulsion eau dans huile comprenant :

   a) une phase huileuse continue, et
   b) au moins une phase aqueuse discontinue dispersée dans ladite phase huileuse ;
   c) au moins un système émulsifiant comprenant :

      i) au moins un ester d'acide gras d'un polyol et
      ii) au moins un polymère d'ester d'acide gras de glycol polyoxyéthylénique ; et

   d) au moins une argile lipophile ; et
   e) au moins une poudre d'élastomère d'organopolysiloxane revêtue avec une résine de silicone.

2. Composition selon la revendication 1, dans laquelle l'ester d'acide gras d'un polyol est le diisostéarate polyhydroxystéarate sébacate de polyglycéryl-4 de formule :

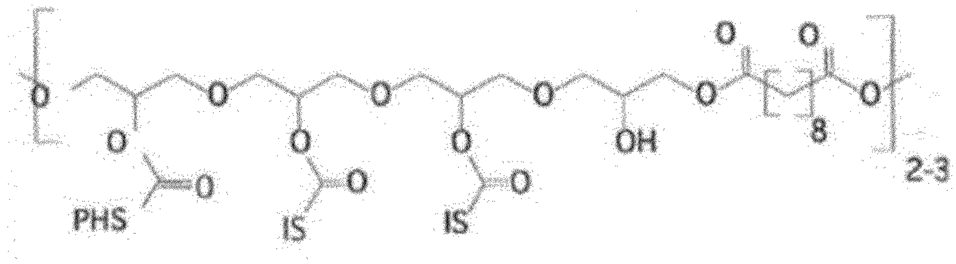

dans laquelle PHS désigne l'acide polyhydroxystéarique et IS désigne l'acide isostéarique, ayant le nom INCI :

Polyglyceryl-4
Diisostearate/Polyhydroxystearate/Sebacate.

**3.** Composition selon la revendication 1, dans laquelle le polymère d'ester d'acide gras de glycol polyoxyéthylénique est le dipolyhydroxystéarate de polyéthylène glycol contenant 30 EO ayant le nom INCI :
PEG-30 Dipolyhydroxystéarate.

**4.** Composition selon l'une quelconque des revendications précédentes, dans laquelle :

i) l'ester d'acide gras d'un polyol est de préférence présent dans la composition à des teneurs dans la plage de 0,1 % à 5 % en poids, plus préférablement de 0,1 % à 2 % en poids et plus particulièrement de 0,1 % à 0,75 % en poids, par rapport au poids total de la composition ; et
ii) le polymère d'ester d'acide gras de glycol polyoxyéthylénique est de préférence présent dans la composition à des teneurs dans la plage de 0,1 % à 5 % en poids, plus préférablement de 0,1 % à 2 % en poids et plus particulièrement de 0,1 % à 1 % en poids, par rapport au poids total de la composition.

**5.** Composition selon l'une quelconque des revendications précédentes, dans laquelle l'argile lipophile est choisie parmi des bentonites qui ont subi une modification hydrophobe et des hectorites qui ont subi une modification hydrophobe, en particulier avec un chlorure d'ammonium quaternaire en $C_{10}$ à $C_{22}$, en particulier le chlorure de stéaralkonium ou le chlorure de distéaryldiméthylammonium.

**6.** Composition selon l'une quelconque des revendications précédentes, dans laquelle les une ou plusieurs argiles lipophiles sont présentes dans la composition à des concentrations de préférence dans la plage de 0,1 % à 5 % en poids et plus préférablement de 0,1 % à 1 % en poids par rapport au poids total de la composition.

**7.** Composition selon l'une quelconque des revendications précédentes, dans laquelle la poudre d'élastomère d'organopolysiloxane est revêtue avec une résine de silsesquioxane, et plus particulièrement choisie parmi :

- celles avec le nom INCI : Vinyl Dimethicone / Methicone Silsesquioxane Crosspolymer ;
- celles avec le nom INCI : Trifluoropropyl Dimethicone / Vinyl Trifluoropropyldimethicone / Silsesquioxane Crosspolymer ;
- celles avec le nom INCI Diphenyl Dimethicone / Vinyl Diphenyl Dimethicone / Silsesquioxane Crosspolymer ;
et des mélanges de celles-ci.

**8.** Composition selon l'une quelconque des revendications précédentes, dans laquelle les une ou plusieurs poudres d'élastomère d'organopolysiloxane revêtues avec une résine de silicone peuvent être présentes à une teneur dans la plage de 0,1 % à 10 % en poids, de préférence de 0,5 % à 5 % en poids, plus particulièrement de 0,5 % à 2 % en poids par rapport au poids total de ladite composition.

**9.** Composition selon l'une quelconque des revendications précédentes, comprenant au moins un additif choisi parmi :

- des agents actifs tels que des vitamines, des agents hydratants ;
- des écrans solaires ;
- des pigments ;

- des charges ;
- des colorants solubles dans l'eau et/ou des colorants liposolubles ;
- et des mélanges de ceux-ci.

**10.** Composition selon l'une quelconque des revendications précédentes, comprenant au moins un pigment, de préférence revêtu avec au moins un composé lipophile ou hydrophobe, en particulier des pigments à revêtement hydrophobe d'oxyde de fer et/ou de dioxyde de titane.

**11.** Composition selon l'une quelconque des revendications précédentes, étant sous la forme d'un fond de teint.

**12.** Procédé de revêtement de matériaux kératiniques, plus particulièrement pour le maquillage et/ou le soin de matériaux kératiniques, tels que la peau, **caractérisé en ce qu'**il comprend l'application sur les matériaux kératiniques d'une composition telle que définie selon l'une quelconque des revendications 1 à 11.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- WO 2016030420 A
- US 20050031580 A
- US 5538793 A
- WO 2007068371 A1
- US 2004175338 A
- EP 847752 A
- EP 0392883 A
- US 5237071 A
- US 5166355 A
- GB 2303549 A
- DE 19726184
- EP 893119 A
- US 5624663 A
- EP 669323 A
- US 2463264 A
- EP 0832642 A
- EP 1027883 A
- EP 1300137 A
- DE 10162844
- WO 9304665 A
- US 4195999 A
- WO 2004006878 A
- WO 2008090066 A
- WO 2011113718 A
- WO 2009027258 A
- WO 2013010590 A
- WO 2013011094 A
- WO 2013011480 A
- WO 2007071584 A
- WO 2009063392 A
- US 6225467 B
- WO 2004085412 A
- WO 06035000 A
- WO 06034982 A
- WO 06034991 A
- WO 06035007 A
- WO 2006034992 A
- WO 2006034985 A
- EP 0841341 A
- EP 0518773 A
- US 4578266 A
- JP H07196946 B
- US 5725882 A
- US 5209924 A
- US 4972037 A
- US 4981903 A
- US 4981902 A
- US 5468477 A
- US 5219560 A
- EP 0388582 A
- US 5246694 A
- EP 0486135 A
- JP H0586984 B
- FR 2679771
- EP 1184426 A

### Non-patent literature cited in the description

- *CHEMICAL ABSTRACTS,* 919803-06-8
- Symmetrical Triazine Derivatives. *IP.COM IPCOM000031257 Journal,* 20 September 2004
- **VAN DE HULST, H.C.** Light Scattering by Small Particles. Wiley, 1957
- **WITUCKI.** A silane primer, chemistry and applications of alkoxy silanes. *Journal of Coatings Technology,* 1993, vol. 65 (822), 57-60
- **C. M. HANSEN.** The three-dimensional solubility parameters. *J. Paint Technol,* 1967, vol. 39, 105